# EUROPÄISCHE PATENTSCHRIFT

(11) **EP 4 703 342 B1**
(45) Veröffentlichungstag und Bekanntmachung des Hinweises auf die Patenterteilung: **09.12.2026**
(21) Anmeldenummer: 24197221.5
(22) Anmeldetag: 29.08.2024
(51) Int. Cl.: C07C 29/149, C07C 31/04, C07C 67/08, C07C 69/06, C07C 51/00, C07C 53/02, C01B 3/00

(54) **VERFAHREN ZUR KATALYTISCHEN ERZEUGUNG VON METHANOL AUS BIOMASSE**
METHOD FOR CATALYTIC PRODUCTION OF METHANOL FROM BIOMASS
PROCÉDÉ DE PRODUCTION CATALYTIQUE DE MÉTHANOL À PARTIR DE BIOMASSE

(43) Veröffentlichungstag der Anmeldung: 04.03.2026
(73) Patentinhaber: OxFA GmbH, 96110 Scheßlitz (DE)
(72) Erfinder: Kohler, Florian, 90419 Nürnberg (DE); Schmidt, Matthias, 91362 Pretzfeld (DE); Dirauf, Martin, 96250 Ebensfeld (DE)
(74) Vertreter: Dr. Gassner & Partner mbB

(56) Entgegenhaltungen:
- HAAGEN VERA ET AL: "Synthesis of methanol by hydrogenolysis of biobased methyl formate using highly stable and active Cu-spinel catalysts in slurry and gas phase reactions", GREEN CHEMISTRY, vol. 25, no. 6, 20 March 2023 (2023-03-20), GB, pages 2338 - 2348, XP093248763, ISSN: 1463-9262, DOI: 10.1039/D2GC04420J

## Beschreibung

Die Erfindung betrifft ein Verfahren und eine Vorrichtung zur katalytischen Erzeugung von Methanol aus Biomasse mittels elektrischem Strom.

Aus der WO 2021/151870 A1 ist ein Verfahren zur katalytischen Erzeugung eines Alkylformiats bekannt, wobei mindestens ein alpha-Hydroxyaldehyd, mindestens eine alpha-Hydroxycarbonsäure, mindestens ein Kohlenhydrat und/oder mindestens ein Glycosid mittels einer Vanadium in der Oxidationsstufe +IV oder +V enthaltenden Vanadium-Sauerstoff-Verbindung oder eines Salzes davon als Katalysator in einer Lösung umgesetzt wird, wobei die Lösung ein Alkanol enthält, wobei das dabei als Reaktionsprodukt entstehende Alkylformiat von mindestens einem dabei entstehenden weiteren Reaktionsprodukt separiert wird, wobei der bei der katalytischen Reaktion reduzierte Katalysator durch Oxidation wieder in seinen Ausgangszustand versetzt wird.

Eine Übersicht über Katalysatoren, die für Veresterungsreaktionen geeignet sind, ist aus Jamil, F. et al., "State-of-the-art catalysts for clean fuel (methyl esters) production-a comprehensive review", J. Phys. Energy 5 (2023) 014005 bekannt.

Aus Haagen, V. et al., "Synthesis of methanol by hydrogenolysis of biobased methyl formate using highly stable and active Cu-spinel catalysts in slurry and gas phase reactions", Green Chem., 2023, 25, 2338-2348 ist ein effizientes zweistufiges Verfahren zur Herstellung von Methanol (MeOH) aus Biomasse bekannt. Dabei wird vorgeschlagen, die Biomasse mittels eines homogenen Polyoxometallat-Katalysators in Anwesenheit von Methanol unter Verwendung von Sauerstoff aus einer Elektrolyse von Wasser in ein Methylformiat (MF)/Ameisensäure (FA)-Gemisch umzuwandeln und das Methylformiat anschließend durch Hydrogenolyse unter Verwendung von Wasserstoff aus einer Elektrolyse von Wasser zu MeOH umzusetzen. Für die Hydrogenolyse werden Cu_{0,9}Al₂O₄-Spinellmaterialien als Katalysatoren vorgeschlagen.

Aus Sang, R. et al. "Methyl formate as a hydrogen energy carrier" Nat Catal 6, 543-550 (2023) ist es bekannt, dass aus Methylformiat mittels eines geeigneten Katalysators durch Dehydrierung Wasserstoff gewonnen werden kann.

Mit einem Angebot von elektrischem Strom, welcher mit zunehmendem Anteil durch erneuerbare Energiequellen erzeugt wird, nehmen Schwankung der im Stromnetz zur Verfügung stehenden Strommenge zu. Um das Stromnetz zu stabilisieren, besteht ein Bedarf an einem sogenannten netzdienlichen Betrieb, d. h. an technischen Verfahren, die dann mehr Strom aus dem Stromnetz beziehen können, wenn er im Überschuss vorliegt und die ihren Strombedarf dann reduzieren können, wenn eine Unterdeckung des Strombedarfs im Netz besteht. Der Überschuss und die Unterdeckung spiegeln sich auch im Strompreis wider, so dass es auch wirtschaftlich sinnvoll ist, dann mehr Strom zu verbrauchen, wenn er im Überschuss vorliegt und den Stromverbrauch dann zu reduzieren, wenn eine Unterdeckung vorliegt. Auch bei netzunabhängig produziertem Strom zur Eigenverwendung - beispielsweise mittels einer Photovoltaikanlage im Inselbetrieb - kann es zu Überdeckungen oder Unterdeckungen gegenüber dem eigenen Bedarf kommen.

In der Publikation des deutschen Bundesumweltamts, Texte 68/2020, Liebich, A. et al., "Systemvergleich speicherbarer Energieträger aus erneuerbaren Energien - Abschlussbericht", ISSN 1862-4804, werden die Ökobilanzen unterschiedlicher Bereitstellungspfade speicherbarer Energieträger aus erneuerbaren Energien analysiert. Bei der Herstellung solcher Energieträger wird entweder von einer Betriebsweise "Vollaststunden Syntheseanlage" ausgegangen, bei der die Anlage während der technisch maximal möglichen jährlichen Betriebsstundenzahl von üblicherweise um die 8000 Stunden in Betrieb ist und dabei rechnerisch von der zugeordneten Stromquelle, z. B. einer Photovoltaikanlage (PV-Anlage), versorgt wird, obwohl dafür notwendige Speicher, z. B. für Strom oder H₂, gar nicht vorhanden sind. Praktisch würde eine solche Betriebsweise einen Betrieb mit einem Mix erneuerbarer Stromquellen oder zeitweise mit dem allgemeinen Strommix erfordern. Alternativ wird von einer Betriebsweise "Volllaststunden Stromquelle" ausgegangen, bei der die Anlage nur während der Betriebsstunden läuft, die den Volllaststunden der zugeordneten Stromquelle entsprechen. Ist die Stromquelle beispielsweise eine PV-Anlage in Marokko sind das durchschnittlich 1729 Volllaststunden pro Jahr. Ein daraus resultierender intermittierender Betrieb kann jedoch nachteilige technische Konsequenzen, wie z. B. eine schnellere Katalysatoralterung oder ein geringer Wirkungsgrad bei Anfahrprozessen, haben. Es wird auch vorgeschlagen, Anlagen zur Herstellung speicherbarer Energieträger nur dann zu betreiben, wenn erneuerbarer Strom im Überschuss vorhanden ist. Dies wird hier als netzdienlicher Betrieb bezeichnet.

Aufgabe der vorliegenden Erfindung ist es, ein alternatives, energieeffizientes und relativ sicheres Verfahren zur katalytischen Erzeugung von Methanol aus Biomasse anzugeben, das einem schwankenden Stromangebot Rechnung trägt. Weiterhin soll eine zur Durchführung eines solchen Verfahrens geeignete Vorrichtung angegeben werden.

Die Aufgabe wird durch die Merkmale der Patentansprüche 1 und 11 gelöst. Zweckmäßige Ausgestaltungen ergeben sich aus den Merkmalen der Patentansprüche 2 bis 10 und 12 bis 15.

Erfindungsgemäß ist ein Verfahren zur katalytischen Erzeugung von Methanol aus Biomasse mittels elektrischem Strom vorgesehen. Die Biomasse ist dabei ein Material, welches mindestens ein alpha-Hydroxyaldehyd, mindestens eine alpha-Hydroxycarbonsäure, mindestens ein Kohlenhydrat und/oder mindestens ein Glycosid umfasst oder daraus besteht. Bei der alpha-Hydroxycarbonsäure kann es sich um Glycolsäure oder Milchsäure und bei dem Kohlenhydrat um ein Monosaccharid, insbesondere mit 5 oder 6 Kohlenstoffatomen, ein Disaccharid, insbesondere mit 12 Kohlenstoffatomen, ein Oligosaccharid oder ein Polysaccharid handeln. Das Monosaccharid kann eine Aldose, insbesondere Glucose oder Xylose, sein. Bei dem Disaccharid kann es sich um Saccharose oder Cellobiose handeln. Das Oligosaccharid kann ein Heterooligosaccharid sein. Bei dem Polysaccharid kann es sich um Stärke, Zellulose, Hemizellulose oder ein Heteropolysaccharid, insbesondere ein Xylan, handeln.

Bei der Biomasse kann es sich um Material biologischen Ursprungs, wie einen, insbesondere nachwachsenden, Rohstoff oder einen nach einer Umsetzung des Rohstoffs daraus hervorgehenden Reststoff handeln. Die Biomasse kann insbesondere pflanzlichen Ursprungs sein. Es ist aber auch möglich, dass sie tierischen Ursprungs ist. So kann es sich dabei beispielsweise um Glycerin handeln, welches aus tierischen Fetten gewonnen wurde. Bei dem Rohstoff kann es sich um Lignocellulose enthaltende Biomasse, wie beispielsweise verholztes Pflanzenmaterial oder Sägemehl, handeln. Bei der Biomasse kann es sich um eine Pflanze, einen Pilz oder Bakterien oder Bestandteile von Pflanzen, Pilzen oder Bakterien, Holz, insbesondere in Form von Holzmehl oder Holzspänen, Papier, insbesondere Altpapier, Algen, Cyanobakterien oder Silage handeln. Alternativ kann sie aus mindestens einer der genannten Substanzen oder einem Gemisch daraus entstanden sein, wie dies z. B. bei Braunkohle oder Torf der Fall ist. Die Biomasse kann auch ein Gemisch aus mindestens zwei der genannten Bestandteile alpha-Hydroxyaldehyd, alpha-Hydroxycarbonsäure, Kohlenhydrat und Glycosid umfassen oder daraus bestehen.

Bei dem Verfahren wird
- in einer ersten Stufe aus Wasser durch Elektrolyse mittels des Stroms O₂ und H₂ erzeugt,
- die Biomasse in einer zweiten Stufe mittels einer wässrigen Lösung eines ersten Katalysators in einem ersten Reaktionsgefäß R1 zu Ameisensäure umgesetzt, wobei der bei der katalytischen Reaktion reduzierte erste Katalysator durch Oxidation wieder in seinen Ausgangszustand versetzt wird, wobei zur Oxidation des reduzierten Katalysators das in der ersten Stufe erzeugte O₂ in die Lösung im ersten Reaktionsgefäß R1 eingebracht wird, wobei der erste Katalysator ein Polyoxometallat-Ion der allgemeinen Formel [PMoₓV_{y}O₄₀]ⁿ⁻ ist, wobei 6 ≤ x ≤ 11, 1 ≤ y ≤ 6 und x + y = 12, [WₓV_{y}O₁₉]ⁿ⁻ ist, wobei x + y = 6, 3 ≤ x ≤ 5 und 1 ≤ y ≤ 3, oder [P₂WₓV_{y}O₆₂]ⁿ⁻ ist, wobei x + y = 18, 12 ≤ x ≤ 17 und 1 ≤ y ≤ 6, oder ein VO²⁺ enthaltendes Salz oder ein [VO₃]⁻ enthaltendes Salz ist, wobei n, x und y jeweils eine ganze Zahl ist, wobei die Lösung mit der darin entstandenen Ameisensäure in ein zweites Reaktionsgefäß R2 überführt wird, wobei der Lösung beim Überführen in das zweite Reaktionsgefäß R2 oder im zweiten Reaktionsgefäß R2 Methanol zugesetzt wird, wobei das zweite Reaktionsgefäß R2 als Rektifikationskolonne ausgebildet ist, in der optional ein eine Veresterung des Methanols mit der Ameisensäure katalysierender saurer zweiter Katalysator enthalten ist, wobei der zweite Katalysator in fester Form als Schüttung oder in flüssiger Form als Säure vorliegt, wobei im zweiten Reaktionsgefäß R2 eine Reaktivdestillation durchgeführt und das dabei entstehende Methylformiat in einen Tank T überführt wird,
- in einer dritten Stufe Methylformiat aus dem Tank T verdampft und in ein drittes Reaktionsgefäß R3 überführt und dort mit dem H₂ aus der ersten Stufe mittels eines eine Hydrierung katalysierenden dritten Katalysators hydriert, wobei durch Hydrogenolyse dampfförmiges Methanol entsteht, welches anschließend aus dem dritten Reaktionsgefäß R3 ausgeleitet und so weit abgekühlt wird, dass das Methanol kondensiert, und
- in einer vierten Stufe der in der zweiten Stufe im zweiten Reaktionsgefäß R2 verbleibende, den ersten Katalysator enthaltende Rückstand der Reaktivdestillation durch Wasserabscheidung konzentriert und dem ersten Reaktionsgefäß R1 zugeführt,
wobei der Strom in einem ersten Verfahrensmodus zur Erzeugung von H₂ in der ersten Stufe eingesetzt wird, welches in der dritten Stufe mit dem Methylformiat zu Methanol umgesetzt wird, das vollständig in das zweite Reaktionsgefäß R2 überführt und in der zweiten Stufe mit der Ameisensäure zu Methylformiat umgesetzt wird, wobei der Strom in einem zweiten Verfahrensmodus zur Erzeugung von H₂ in der ersten Stufe eingesetzt wird, welches in der dritten Stufe mit dem Methylformiat zu Methanol umgesetzt wird, das in das zweite Reaktionsgefäß R2 überführt und in der zweiten Stufe mit der Ameisensäure zu Methylformiat umgesetzt wird und außerdem in der ersten Stufe zum Erzeugen von zusätzlichem H₂ eingesetzt wird, wobei dieses zusätzliche H₂ in der dritten Stufe zum Erzeugen von zusätzlichem Methanol eingesetzt wird, welches aus dem Verfahren ausgeschleust wird. Das Verfahren wird abwechselnd im ersten und im zweiten Verfahrensmodus betrieben.

Bei dem erfindungsgemäßen Verfahren wird sowohl im ersten als auch zweiten Verfahrensmodus in der ersten Stufe im Idealfall stets so viel H₂ durch Elektrolyse erzeugt, dass die in der dritten Stufe des Verfahrens erzeugte Menge an Methanol ausreicht, um die in der zweiten Stufe des Verfahrens kontinuierlich entstehende Menge an Ameisensäure zu Methylformiat umzusetzen. Das Besondere an dem erfindungsgemäßen Verfahren ist, dass die zweite Stufe des Verfahrens idealerweise unabhängig vom Verfahrensmodus kontinuierlich mit einem stets gleichbleibenden Umsatz an Biomasse betrieben werden kann, während die dritte Stufe des Verfahrens, das heißt die Hydrierung des Methylformiats durch die Wahl des zweiten Verfahrensmodus an ein erhöhtes Stromangebot und damit eine höhere durch die Elektrolyse bereitstellbare H₂-Menge angepasst werden kann. Dies ist möglich, weil das im Tank T befindliche Methylformiat als Puffer dient. Der Tank T füllt sich im ersten Verfahrensmodus mit Methylformiat, weil aus einem Molekül Methylformiat durch Hydrierung zwei Moleküle Methanol entstehen, pro Molekül des Methylformiats aber nur ein Molekül Ameisensäure und ein Molekül Methanol benötigt werden. Dadurch entsteht im ersten Verfahrensmodus mehr Methylformiat als zur Aufrechterhaltung der Reaktion im zweiten Reaktionsgefäß erforderlich ist.

Im ersten Verfahrensmodus wird, beispielsweise bei geringem Stromangebot im Verhältnis zum Strombedarf, lediglich so viel Methylformiat hydriert, dass die daraus entstehende Menge an Methanol ausreicht, um die entstehende Menge Ameisensäure umzusetzen. Steht Strom im Verhältnis zum Strombedarf im Überschuss zur Verfügung, kann im zweiten Verfahrensmodus durch Hydrierung von Methylformiat aus dem Tank T so viel Methanol erzeugt werden, dass der nicht zur Veresterung mit Ameisensäure zur Erzeugung von Methylformiat erforderliche Anteil davon ausgeschleust werden kann. Sofern der Füllstand des Tanks T mit Methylformiat auf dieses Weise nicht ausreichend abgebaut werden kann, kann auch Methylformiat als Produkt aus dem Verfahren ausgeschleust werden. Die entsprechende Steuerung des Verfahrens kann über eine Regeleinheit erfolgen, die z. B. den Füllstand des Tanks T überwacht und bei Überschreitung eines zuvor festgelegten ersten Füllstands vom ersten in den zweiten Verfahrensmodus umschaltet, damit der Füllstand abnimmt und bei unterschreiten eines zuvor festgelegten zweiten Füllstands in den ersten Verfahrensmodus umschaltet. Der zweite Füllstand liegt dabei stets unterhalb des ersten Füllstands und je weiter unterhalb er liegt, desto größer ist die Pufferwirkung des Tanks T, die darin besteht, dass Strom dann vermehrt verbraucht werden kann, wenn er im Überschuss zur Verfügung steht.

Das Umschalten zwischen dem ersten und dem zweiten Verfahrensmodus erfolgt im einfachsten Fall dadurch, dass die Leistung eines für die Durchführung der Elektrolyse vorgesehenen Elektrolyseurs in der ersten Stufe des Verfahrens hochreguliert wird, so dass mehr H₂ erzeugt wird und, insbesondere gleichzeitig, die Menge des in der dritten Stufe verdampften und dem dritten Reaktionsgefäß zugeführten Methylformiats hochreguliert und so an die erhöhte H₂-Menge angepasst wird. Das H₂ wird dann in der dritten Stufe mit dem Methylformiat zu mehr Methanol umgesetzt als zur Aufrechterhaltung des Verfahrens erforderlich ist. Ein späteres Umschalten vom zweiten Verfahrensmodus in den ersten Verfahrensmodus erfolgt in umgekehrter Weise, d. h. dadurch, dass die Leistung des Elektrolyseurs in der ersten Stufe des Verfahrens herunterreguliert wird, so dass weniger H₂ erzeugt wird und, insbesondere gleichzeitig, die Menge des in der dritten Stufe verdampften und dem dritten Reaktionsgefäß zugeführten Methylformiats herunterreguliert und so an die verringerte H₂-Menge angepasst wird. Das H₂ wird dann in der dritten Stufe mit dem Methylformiat zu so viel Methanol umgesetzt wie zur Aufrechterhaltung des Verfahrens erforderlich ist. Bei dem Elektrolyseur kann es sich beispielsweise um einen Protonen-Austausch-Membran-Elektrolyseur handeln.

Die Überwachung des Füllstands des Tanks T ist optional, weil ein Überschuss an erzeugtem Methylformiat auch passiv, zum Beispiel durch das Vorsehen eines Überlaufs, abgeleitet werden kann. Die Regeleinheit kann ansonsten vor allem steuern, welcher Anteil des erzeugten Methanols dem zweiten Reaktionsgefäß R2 zur Veresterung mit der Ameisensäure zugeführt wird und welcher Anteil davon als Produkt aus dem Verfahren ausgeleitet wird. Die Menge an Methanol, die dem zweiten Reaktionsgefäß R2 zur Veresterung mit der Ameisensäure zugeführt wird, wird dabei möglichst konstant gehalten. Bei dem gesamten Verfahren sollte die Umsetzung der Biomasse zur Ameisensäure und der Ameisensäure zu Methylformiat möglichst mit konstantem Massendurchsatz erfolgen und nur die Menge des in der ersten Stufe erzeugten Wasserstoffs und des zu Methanol umgesetzten Methylformiats durch die Wahl des ersten oder zweiten Verfahrensmodus variiert werden. Alternativ zur Steuerung des Anteils des Methanols, der dem zweiten Reaktionsgefäß R2 zugeführt und des Anteils, der ausgeleitet wird ist auch eine passive Regelung möglich. Dazu kann ein weiterer Tank zur Aufnahme des in der dritten Stufe des Verfahrens erzeugten Methanols vorgesehen sein. Daraus kann dem zweiten Reaktionsgefäß mittels einer Pumpe eine konstante Menge Methanol pro Zeiteinheit zur Veresterung mit der Ameisensäure zugeführt werden. Das Ausschleusen des Methanols, das nicht für die Veresterung benötigt wird, kann entweder passiv durch einen im weiteren Tank vorgesehenen Überlauf oder mittels einer, insbesondere durch den Füllstand des weiteren Tanks regulierten, weiteren Pumpe erfolgen.

Die Wahl zwischen dem ersten und dem zweiten Verfahrensmodus kann beispielsweise in Abhängigkeit von einer in einem System zur Verfügung stehenden Strommenge im Verhältnis zu dem in dem System bestehenden Strombedarf erfolgen. Bei dem Strom kann es sich um Strom ausschließlich aus einem öffentlichen Stromnetz, Strom aus einem öffentlichen Stromnetz in Kombination mit Strom aus einer Photovoltaikanlage, Windkraftanlage oder sonstigen Anlage mit schwankender Stromerzeugung oder um Strom aus einem geschlossenen Stromnetz, beispielsweise bei einer eigenen Energieerzeugung mittels einer Photovoltaikanlage oder Windkraftanlage in Kombination mit einem Kleinkraftwerk, handeln. Ist das Verhältnis "zur Verfügung stehende Strommenge : Strombedarf" kleiner oder gleich 1 wird das Verfahren vorzugsweise im ersten Verfahrensmodus betrieben, anderenfalls, d. h. wenn das Verhältnis größer als 1 ist, im zweiten Verfahrensmodus. Das erfindungsgemäße Verfahren ist besonders effektiv, wenn es jeweils für längere Zeit im ersten und zweiten Verfahrensmodus durchgeführt wird. Um bei einem um den Wert 1 herum schwankenden Verhältnis zwischen zur Verfügung stehender Strommenge und Strombedarf nicht so häufig zwischen dem ersten und zweiten Verfahrensmodus wechseln zu müssen, kann zur Pufferung der zur Verfügung stehenden Strommenge ein Akkumulator oder ein Batteriesystem vorgesehen sein. Das erfindungsgemäße Verfahren ermöglicht eine sehr energieeffiziente und dennoch netzdienliche Erzeugung von Methanol.

Ein wesentlicher Unterschied des Verfahrens gegenüber dem von Haagen et al. vorgeschlagenen Verfahren besteht darin, dass das Methanol nicht bereits vor oder bei der katalytischen Umsetzung der Biomasse und der räumlich und zeitlich davon nicht getrennten Oxidation des dabei reduzierten Katalysators zugesetzt wird. Im Gegensatz dazu wird das Methanol hier erst nach Überleitung der Lösung bzw. Reaktionsflüssigkeit in das zweite Reaktionsgefäß R2 zugesetzt. Dadurch wird vermieden, dass bei der Oxidation des Katalysators durch Begasung mit O₂ aus dem Methanol und dem O₂ ein reaktionsfähiges und insbesondere explosives Gasgemisch entsteht. Das erfindungsgemäße Verfahren ist dadurch wesentlich ungefährlicher.

Die Oxidation des Katalysators mit Sauerstoff aus der Elektrolyse in der ersten Stufe des Verfahrens hat gegenüber der Oxidation mit in Luft enthaltenem Sauerstoff den Vorteil, dass für eine effiziente Oxidation ein deutlich geringerer Gesamtdruck und damit auch ein deutlich geringerer Energieaufwand für die Bereitstellung dieses Gesamtdrucks erforderlich ist. Für die Effizienz der Oxidation ist nämlich lediglich der Sauerstoffpartialdruck entscheidend. Um einen Sauerstoffpartialdruck von 5 bar mittels Luft zu erreichen, wäre beispielsweise ein Luftdruck von 23,8 bar erforderlich. Zumindest das erste Reaktionsgefäß R1 kann daher weniger druckresistent und damit günstiger ausgelegt werden. Außerdem ist das Verfahren durch die Bereitstellung von Sauerstoff aus der Elektrolyse wirtschaftlicher zu betreiben, weil eine Sauerstoffabsonderung aus Luft mittels Druckwechseladsorption entfallen kann oder zumindest nur einen Teil des Sauerstoffbedarfs des Verfahrens decken müsste, sofern der durch die Elektrolyse gewonnene Sauerstoff nicht ausreicht, um den gesamten in der zweiten Stufe des Verfahrens reduzierten Katalysator zu oxidieren.

Der zweite Katalysator ist optional, weil er für die Durchführung des Verfahrens nicht erforderlich ist, denn der in der Lösung vorliegende erste Katalysator ist von Natur aus sauer und katalysiert dadurch bereits die Veresterung von Ameisensäure und Methanol zu Methylformiat im zweiten Reaktionsgefäß R2. Solange noch nicht veresterte Ameisensäure im zweiten Reaktionsgefäß R2 vorliegt, wirkt diese zusätzlich katalytisch bei der Veresterung. Ist jedoch im zweiten Reaktionsgefäß R2 der zweiter Katalysator in fester Form enthalten, ist eine weitere Besonderheit des Verfahrens, dass die Veresterung von Ameisensäure und Methanol zu Methylformiat im zweiten Reaktionsgefäß R2 katalytisch durch eine Kombination des in der Lösung vorliegenden ersten Katalysators mit dem in fester Form vorliegenden sauren zweiten Katalysator bewirkt wird. Dadurch wird die Effizienz der Veresterung gegenüber der lediglich durch den ersten Katalysator bewirkten Veresterung deutlich gesteigert und das Methylformiat wird in hoher Reinheit erhalten.

Eine weitere Besonderheit des erfindungsgemäßen Verfahrens besteht in dessen großer Wirtschaftlichkeit und dem geringen Ressourcenbedarf. So kann beispielsweise die bei der Oxidation der Biomasse im ersten Reaktionsgefäß R1 und bei der Hydrierung im dritten Reaktionsgefäß R3 frei werdende Wärme, insbesondere vollständig, genutzt werden, um den Wärmebedarf der Reaktivdestillation im zweiten Reaktionsgefäß R2 und gegebenenfalls bei der Wasserabscheidung in der vierten Stufe des Verfahrens zu decken oder zumindest einen überwiegenden Teil dazu beizutragen. Die insgesamt bei dem Verfahren frei werdende Wärme hängt von der dafür eingesetzten Biomasse ab und kann sogar die für die Durchführung des Verfahrens erforderliche Wärme übersteigen, so dass Wärme aus dem Verfahren ausgeschleust und anderweitig genutzt werden kann, beispielsweise bei einer Nahwärmeversorgung oder zur Erzeugung von Strom. Dieser Strom kann für die Elektrolyse in der ersten Stufe des erfindungsgemäßen Verfahrens oder anderweitig genutzt werden kann. Wird beispielsweise Glycerin als Biomasse eingesetzt, ergibt sich - bezogen auf den für die Elektrolyse eingesetzten Strom und einem unteren Heizwert des ausgeschleusten Methanols von 5,47 kWh/kg - eine theoretische Energieeffizienz von 87,5%. Wird auch der Heizwert des Glycerins mit eingerechnet ergibt sich noch immer eine theoretische Energieeffizienz von 43,2%.

Weiterhin kann bei einem kontinuierlichen Betrieb des Verfahrens der gesamte für die Elektrolyse erforderliche Wasserbedarf durch die Wasserabscheidung in der vierten Stufe des Verfahrens gedeckt werden, wenn der Wassergehalt der Biomasse hoch genug ist. Das in der vierten Stufe durch die Wasserabscheidung abzuscheidende Wasser stammt aus der Biomasse und den Reaktionen im ersten Reaktionsgefäß R1 und im zweiten Reaktionsgefäß R2 in der zweiten Stufe des Verfahrens.

Das bei Haagen et al. diskutierte und durch den Einsatz der Spinellmaterialien als Katalysatoren gelöste Problem der Korrosion und des Auslaugens der üblichen Hydrogenolysekatalysatoren durch Ameisensäure tritt bei dem erfindungsgemäßen Verfahren nicht auf, weil die Ameisensäure im ersten Reaktionsgefäß R1 und zweiten Rektiongefäß R2 von dem die Hydrierung katalysierenden dritten Katalysator im dritten Reaktionsgefäß R3 räumlich getrennt ist. Dadurch kann für die Hydrierung bzw. Hydrogenolyse in der dritten Stufe des Verfahrens ein wesentlich günstiger Katalysator, wie beispielsweise ein CuO/Cr₂O₃-Katalysator oder ein CuO/MgO/ZnO/Al₂O₃-Katalysator, eingesetzt werden. Um eine gegebenenfalls aus Methylformiat durch Hydrolyse erfolgende Entstehung von Ameisensäure zumindest weitgehend zu unterbinden kann das in der zweiten Stufe entstehende Methylformiat mit einem Absorbermaterial zum Binden von Wasser, beispielsweise im Tank, in Kontakt gebracht werden. Bei dem Absorbermaterial kann es sich zum Beispiel um ein Molekularsieb, insbesondere aus Zeolith, oder ein Silikagel handeln.

Durch das Vorsehen eines Tanks **T,** in dem erzeugtes Methylformiat zwischengelagert werden kann, kann bei hohem Stromangebot die Leistung eines Elektrolyseurs und damit die erzeugte H₂-Menge in der ersten Stufe des Verfahrens erhöht werden und das kontinuierlich produzierte und das zwischengelagerte Methylformiat mit dem dann in erhöhter Menge zur Verfügung stehenden H₂ in der dritten Stufe des Verfahrens durch Hydrierung und Hydrogenolyse zu Methanol umgesetzt werden. Auf diese Weise kann die zwischengelagerte Menge an Methylformiat als Puffer dienen, um Strom dann aufzunehmen bzw. vermehrt zu verbrauchen, wenn er im Überschuss zur Verfügung steht.

Bei einer Ausgestaltung des Verfahrens wird in der zweiten Stufe des Verfahrens mindestens eine solche Menge an Methanol zugesetzt, dass sämtliche Ameisensäure im zweiten Reaktionsgefäß R2 damit zu Methylformiat umgesetzt wird.

Ein Überschuss an Methanol im zweiten Reaktionsgefäß ist unschädlich. Überschüssiges Methanol wird in der vierten Stufe des Verfahrens üblicherweise weitgehend zusammen mit dem Wasser abgeschieden. Es ist auch möglich, beispielsweise mittels einer zweistufigen Destillation, Wasser und Methanol gesondert abzuscheiden, um dadurch zusätzlich Methanol zu gewinnen, um es als Produkt aus dem Verfahren auszuschleusen oder dem zweiten Reaktionsgefäß R2 zur Veresterung mit der Ameisensäure zuzuführen. Eine gegebenenfalls verbleibende geringe Menge an Methanol wird danach zusammen mit der Lösung des ersten Katalysators nach der vierten Stufe des Verfahrens wieder der zweiten Stufe des Verfahrens im ersten Reaktionsgefäß R1 zugeführt. Eine geringe Menge an Methanol erhöht gemäß der WO 2021/151870 A1 die Selektivität des Verfahrens. Die damit in das erste Reaktionsgefäß R1 gelangende Menge an Methanol ist dabei jedoch nicht so hoch, dass dadurch mit dem eingebrachten Sauerstoff ein reaktives Gemisch entsteht.

Bei einer weiteren Ausgestaltung des Verfahrens wird am Beginn einer Durchführung des Verfahrens in der zweiten Stufe Methanol von außen, d.h. nicht aus der dritten Stufe des Verfahrens, zugeführt, da Methanol aus der dritten Stufe des Verfahrens am Beginn der Durchführung des Verfahrens nicht oder nicht in ausreichender Menge zur Verfügung steht. Der Beginn des Verfahrens umfasst den Start und die Anlaufphase bis in der dritten Stufe des Verfahrens so viel Methanol zur Verfügung gestellt werden kann, dass es zur Umsetzung der in der zweiten Stufe des Verfahrens erzeugten Ameisensäure ausreichend ist. Bei der Ausgestaltung des Verfahrens wird Methanol nur so lange und nur in einer solchen Menge von außen zugeführt, wie es erforderlich ist, damit insgesamt in der zweiten Stufe zusammen mit dem aus der dritten Stufe stammenden und in das zweite Reaktionsgefäß R2 überführten Methanol genug Methanol zur Verfügung steht, um damit sämtliche Ameisensäure im zweiten Reaktionsgefäß R2 zu Methylformiat umzusetzen.

Die Menge des für die Oxidation des Katalysators erforderlichen Sauerstoffs hängt von der Biomasse ab, welche für das Verfahren eingesetzt wird. Kann der daraus resultierende Sauerstoffbedarf für die Oxidation des Katalysators nicht durch den bei der Elektrolyse entstehenden Sauerstoff gedeckt werden, kann dieser zusätzlich auch durch Zufuhr von Luft oxidiert werden. Dies hat jedoch den Nachteil, dass zum Erreichen eines für eine effektive Oxidation geeigneten Sauerstoffpartialdrucks der Gesamtdruck, mit der die Luft in die zweite Stufe eingebracht werden muss, deutlich höher ist als wenn reiner Sauerstoff oder zumindest mit Sauerstoff angereicherte Luft in das erste Reaktionsgefäß R1 eingebracht werden würde. Das erste Reaktionsgefäß müsste dann deutlich druckresistenter ausgeführt sein, wodurch die Anlagenkosten erhöht werden würden.

Bei einer alternativen Ausgestaltung des Verfahrens wird daher mittels Druckwechseladsorption O₂ aus Luft separiert oder eine O₂-angereicherte Luft erzeugt und dieses O₂ oder diese O₂-angereicherte Luft zur Oxidation des bei der katalytischen Reaktion reduzierten ersten Katalysators zusätzlich zum in der ersten Stufe erzeugten O₂ in die Lösung im ersten Reaktionsgefäß R1 eingebracht. Es hat sich gezeigt, dass der Energieaufwand für die Druckwechseladsorption (PSA) zur Separierung oder Anreicherung von O₂ den Energieaufwand für die Kompression von Luft, um diese mit erhöhtem Druck in der zweiten Stufe des Verfahrens einzubringen, nicht oder allenfalls unwesentlich übersteigt. Alternativ oder zusätzlich kann zur Oxidation des bei der katalytischen Reaktion reduzierten ersten Katalysators zusätzlich zum in der ersten Stufe erzeugten Sauerstoff Luft in die Lösung im ersten Reaktionsgefäß R1 eingebracht werden.

Die Oxidation mittels des Sauerstoffs kann bei einem Sauerstoffpartialdruck im Bereich von 1 bar bis 30 bar, insbesondere 5 bar bis 20 bar, insbesondere 5 bar bis 10 bar, erfolgen. Der in der ersten Stufe erzeugte Sauerstoff kann dazu mit einem solchen Druck in die Lösung im ersten Reaktionsgefäß R1 eingebracht werden. Zur Oxidation kann die Lösung, beispielsweise in einem statischen Mischer oder durch heftiges Rühren, mit dem Sauerstoff beaufschlagt werden.

Für das Verfahren hat es sich als effizient erwiesen, wenn das Umsetzen der Biomasse zu Ameisensäure bei einer Temperatur im Bereich von 50 °C bis 150 °C, insbesondere 80 °C bis 145 °C, insbesondere 90 °C bis 140 °C, insbesondere 100 °C bis 135 °C, insbesondere 110 °C bis 130 °C, erfolgt.

Ist der Katalysator eine in flüssiger Form vorliegende Säure, kann es sich dabei beispielsweise um Salzsäure oder Schwefelsäure handeln. Der zweite Katalysator kann ZnO/ZrO₂ oder ein, insbesondere poröser, insbesondere mesoporöser, geträgerter, insbesondere Siliziumdioxid-geträgerter, Diarylammonium-Katalysator, insbesondere in Form eines Diphenylammoniumsalzes, insbesondere Diphenylammonium-triflat, ein saurer Kationenaustauscher, wie z.B Amberlyst^{®} 15, insbesondere Amberlyst^{®} 15H, Amberlyst^{®} 70 oder Dowex^{®} 50WX2 jeweils von The Dow Chemical Company oder ein saurer Zeolith, wie z. B. Zeolith HZSM-5 oder Zeolith HY, oder ein Polystyrolsulfonat, eine geträgerte, insbesondere von Siliziumdioxid, Aktivkohle oder porösem Glas geträgerte, Heteropolysäure, insbesondere Wolframatophosphorsäure, insbesondere siliziumdioxid-geträgerte Wolframatophosphorsäure, 2-[1-[Difluor[(trifluorethenyl)oxy]methyl]-1,2,2,2-tetrafluo-rethoxy]-1,1,2,2-tetrafluorethansulfonsäure (Nafion^{®}) oder mesoporöses Silika sein, wenn es ein in fester Form vorliegender zweiter Katalysator ist. Das mesoporöse Silika kann SBA-15 von Merck KGaA (Sigma-Aldrich, Artikelnummer 914614), d. h. Silika mit einer Partikelgröße bis maximal 150 µm, einer Porengröße von 10 nm, und hexagonaler Porenmorphologie oder MCM-41 von Merck KGaA (Sigma-Aldrich, Artikelnummer 643645), d. h. mesostrukturiertes Siliziumdioxid sein.

Der die Hydrierung bei der dritten Stufe des Verfahrens katalysierende dritte Katalysator kann ein aus mindestens einem Metall in elementarer oder oxidierter Form bestehender oder ein solches Metall enthaltender und in fester Form vorliegender Hydrierungskatalysator sein. Dabei kann es sich beispielsweise um Pt , Pd, Rh, Ru, Ni, Co, Fe und Cu oder eine Legierung oder Mischung davon, insbesondere jeweils in poröser oder feinverteilter Form oder auf einem porösen Träger, insbesondere auf Aktivkohle, auf Aluminiumoxid oder auf Silika jeweils mit einem Metallanteil von 0,5 - 10 Gew.-%, insbesondere 0,5 - 5 Gew.-%, handeln. Mögliche Katalysatoren sind beispielsweise Platinschwarz, Platin(IV)-oxid, Palladiumschwarz, kolloides Palladium, Palladium(II)-oxid, Palladium auf Calciumcarbonat oder Bariumsulfat, ein Lindlar-Katalysator, ein Nishimura-Katalysator, der ein Rhodium-Platin-Mischoxid ist, Nickel auf Kieselgur, ein Raney-Nickel-Katalysator, ein NiSAT^{®} Katalysator der Firma Claraint, Kupferchromit (CuCr₂O₄), ein Adkins-Katalysator, ein HyMax^{™} Katalysator der Firma Clariant, ein Kupfer-Zink, Kupfer-Alumina oder Kupfer-Bismuth Katalysator, insbesondere ein HySAT^{®} Katalysator der Firma Clariant, oder Raney-Cobalt. Insbesondere kann der die Hydrierung katalysierende Katalysator ein Cu_{0.9}Al₂O₄ Spinel-Typ-Katalysator, wie er aus Haagen, V. et al., Green Chem., 2023, 25, 2338-2348 bekannt ist, ein CuO/Cr₂O₃-Katalysator, ein CuO/MgO/ZnO/Al₂O₃-Katalysator, ein Cu-ZnO₂-Katalysator, ein Cu-SiO₂-Katalysator, insbesondere ein unter Verwendung des Ammoniak-Verdampfungsverfahrens Verfahrens hergestellter Cu-SiO₂-AE-Katalysator (AE = ammoniaevaporation), wie er in Wu, J. et al., "A Cu-SiO2 Catalyst for Highly Efficient Hydrogenation of Methyl Formate to Methanol" Catalysts 2023, 13, 1038 beschrieben ist, ein Raney-Kupfer Katalysator oder ein Kupfer-Aluminium-Legierung-Katalysator, wie er in Huang, X. et al., Catalysis Today, Volumes 93-95, 1 September 2004, Pages 113-119 beschrieben ist, sein.

Die Wasserabscheidung in der vierten Stufe des Verfahrens kann durch Entspannungsverdampfung oder eine sonstige Verdampfung, insbesondere mit nur einer theoretischen Trennstufe, d. h. ohne einen Rücklauf, erfolgen. Dazu kann beispielsweise ein Kurzwegverdampfer, Dünnschichtverdampfer, Fallfilmverdampfer, Kesselverdampfer oder Naturumlaufverdampfer eingesetzt werden. Das dabei verdampfte Wasser kann kondensiert werden, um es in der ersten Stufe des Verfahrens zur Elektrolyse einzusetzen. Die beim Kondensieren gewonnene Wärme kann mittels eines Wärmetauschers für eine weitere Verdampfung zur Wasserabscheidung in der vierten Stufe des Verfahrens, insbesondere unter reduziertem Druck und damit geringerem Wärmebedarf, verwendet werden. Alternativ kann Wasser auch durch Pervaporation, d. h. mittels einer Membran, abgeschieden werden. Bei der Pervaporation durchdringt Wasser oder ein Wasser-Methanol-Gemisch die Membran und verdampft auf der der Lösung abgewandten Seite der Membran. Auf der der Lösung abgewandten Seite der Membran kann dazu ein Unterdruck anliegen und/oder ein Strippgas, z. B. Luft, vorbeigeströmt werden. Bei der Membran kann es sich um eine Hohlfasermembran handeln. Alternativ kann die Wasserabscheidung auch mittels einer Rektifikation oder zweistufigen Destillation erfolgen, mittels der auch eine Abscheidung von in dem Rückstand noch enthaltenem Methanol erfolgen kann.

Die Menge des bei der Wasserabscheidung in der vierten Stufe des Verfahrens abgeschiedenen Wassers kann so an einen Wassergehalt der Biomasse angepasst werden, dass umso mehr Wasser abgeschieden wird, je höher der Wassergehalt der Biomasse ist. Bei einer Biomasse, deren Wassergehalt hoch genug ist, kann das gesamte erfindungsgemäße Verfahren ohne während des Verfahrens von außen zugeführtes Wasser durchgeführt werden. Das wird dadurch begünstigt, dass bei den Reaktionen im ersten Reaktionsgefäß R1 und im zweiten Reaktionsgefäß R2 in der zweiten Stufe des Verfahrens jeweils Wasser freigesetzt wird.

Im ersten Verfahrensmodus kann das in der ersten Stufe erzeugte H₂ teilweise durch H₂ ergänzt oder ersetzt werden, das durch Dehydrierung des in der zweiten Stufe erzeugten Methylformiats mittels eines Dehydrierungskatalysators erzeugt wird. Das teilweise Ersetzen kann bedeuten, dass mittels Strom weniger H₂ durch Elektrolyse erzeugt wird und das dadurch fehlende H₂ durch H₂ aus der Dehydrierung von Methylformiat ersetzt wird. Es kann aber auch bedeuten, dass in der ersten Stufe für eine begrenzte und sich nicht über die gesamte Zeit der Durchführung des Verfahrens im ersten Verfahrensmodus erstreckende Zeit H₂ ausschließlich durch die Dehydrierung von Methylformiat gewonnen wird. Dadurch kann das Verfahren fortgeführt werden, wenn für eine begrenzte Zeit kein oder kaum Strom zur Verfügung steht.

Die katalytische Dehydrierung von Methylformiat erfolgt gemäß folgender Reaktionsgleichung:

CH₃OOCH + 2 H₂O -> 4 H₂ + 2 CO₂

Sie erfolgt bevorzugt und besonders effektiv bei einer Temperatur im Bereich von 70 °C bis 100 °C unter einem Druck im Bereich von 4 bis 40 bar, insbesondere 20 bis 40 bar. Da aus einem Molekül Methylformiat durch Hydrierung mit zwei Molekülen H₂ zwei Moleküle Methanol entstehen, aus einem Molekül Methylformiat und 2 Molekülen Wasser durch Dehydrierung aber 4 Moleküle H₂, kann durch die Dehydrierung genug Wasserstoff gewonnen werden, um eine Methanolmenge zu erzeugen, die zur Aufrechterhaltung der Erzeugung des Methylformiats erforderlich ist. Insgesamt kann doppelt so viel Methylformiat erzeugt werden, wie für die Wasserstofffreisetzung durch Dehydrierung von Methylformiat verbraucht wird. Als Katalysator für die Dehydrierung des Methylformiats kann einer der aus Sang, R. et al. "Methyl formate as a hydrogen energy carrier" Nat Catal 6, 543-550 (2023) bekannten Katalysatoren eingesetzt werden. Das kann beispielsweise ein Ruthenium-Pincer-Komplex, wie einer der im Folgenden dargestellten sein:

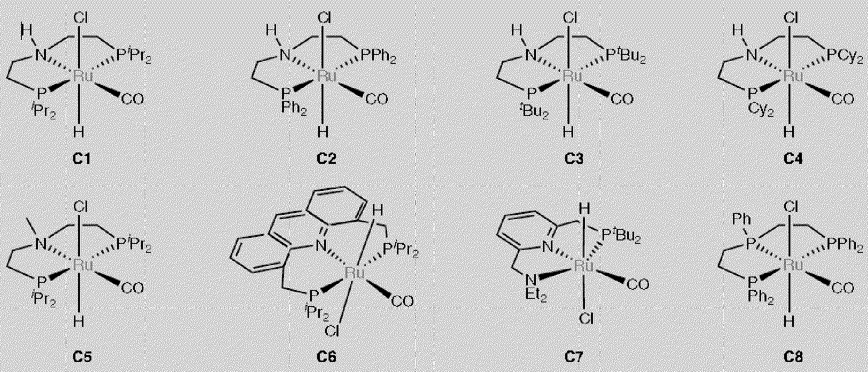

Als besonders geeignet haben sich davon die mit C1 bis C5 bezeichneten Katalysatoren erwiesen.

**In** der vierten Stufe des Verfahrens abgeschiedenes Wasser kann der Elektrolyse in der ersten Stufe und/oder der Dehydrierungsreaktion des Methylformiats zugeführt werden. Bevor es der Elektrolyse in der ersten Stufe des Verfahrens zugeführt wird, kann das Wasser hinsichtlich des pH-Werts durch Zusatz einer, insbesondere anorganischen, Base oder, insbesondere anorganischen, Säure an das in der ersten Stufe des Verfahrens bei der Elektrolyse eingesetzte Wasser angeglichen werden. Auch eine Angleichung des abgeschiedenen Wassers an das bei der Elektrolyse eingesetzte Wasser hinsichtlich dessen Wasserhärte und dessen Leitfähigkeit, insbesondere durch Hindurchleiten durch einen oder mehrere Ionentauscher, und/oder hinsichtlich der darin gelösten Gase, insbesondere durch eine Entgasung mittels Unterdruck oder mittels einer gasdurchlässigen flüssigkeitsundurchlässigen Membran, ist möglich. Sofern in dem in der vierten Stufe abgeschiedene Wasser organische Bestandteile enthalten sind, kann eine weitere Angleichung an das in der ersten Stufe des Verfahrens bei der Elektrolyse eingesetzte Wasser dadurch erfolgen, dass diese organischen Bestandteile, beispielsweise mittels Umkehrosmose, aus dem Wasser abgeschieden werden, bevor das Wasser der Elektrolyse in der erstem Stufe des Verfahrens zugeführt wird. Die Anpassung des Wassers an des für die Elektrolyse eingesetzte Wasser wird in der Technik üblicherweise als Konditionierung bezeichnet. Dadurch kann erforderlichenfalls verhindert werden, dass eine in der ersten Stufe des Verfahrens eingesetzte Elektrolyseeinheit geschädigt wird oder die Elektrolyse durch die Zuführung des in der vierten Stufe des Verfahrens abgeschiedenen Wassers ineffizient wird. Eine Elektrolyse mittels eines Protonen-Austausch-Membran-Elektrolyseurs erfordert beispielsweise deionisiertes Wasser, welches einen spezifischen elektrischen Widerstand von mehr als 1 MΩ*cm bei 20 °C aufweist. Um einen solch hohen spezifischen elektrischen Widerstand zu erreichen, kann das in der vierten Stufe des Verfahrens abgeschiedene Wasser z. B. mittel Umkehrosmose oder mittels eines Ionentauschers konditioniert werden.

Wenn das Verfahren möglichst einfach durchgeführt werden soll und der Wasserverbrauch für die Elektrolyse auf Grund äußerlicher Umstände keine oder zumindest keine wesentliche Rolle spielt, kann für die Elektrolyse auch stetig Wasser von außen zugeführt werden. Dann kann die Konditionierung entfallen. Wenn die Wasserabscheidung durch Verdampfen erfolgt und auch auf die Gewinnung von Wärme durch Kondensation des verdampften Wassers verzichtet werden kann, kann auch die Kondensation des Wassers entfallen und das Verfahren dadurch weiter vereinfacht werden.

Um eine möglichst effiziente Nutzung der bei dem erfindungsgemäßen Verfahren frei werdenden Wärme zu ermöglichen, kann im ersten Reaktionsgefäß R1 in der zweiten Stufe entstehende Wärme mittels eines ersten Wärmetauschers und im dritten Reaktionsgefäß R3 in der dritten Stufe des Verfahrens entstehende Wärme mittels eines zweiten Wärmetauschers abgeführt und dem zweiten Reaktionsgefäß R2 mittels eines dritten Wärmetauschers und/oder der Wasserabscheidung in der vierten Stufe des Verfahrens, sofern dafür ein Wärmebedarf besteht, mittels eines vierten Wärmetauschers zugeführt werden.

Um die Sicherheit des Verfahrens weiter zu erhöhen, kann die im ersten Reaktionsgefäß R1 entstehende, die Ameisensäure enthaltende Lösung vor deren Überführung in das zweite Reaktionsgefäß R2 entgast werden, um in der Lösung gelösten Sauerstoff zu entfernen und dessen Freisetzung und Kontakt mit dem Methanol im zweiten Reaktionsgefäß R2 und damit die Entstehung eines zündfähigen Gemischs sicher zu verhindern. Das Entgasen kann beispielsweise mittels einer gasdurchlässigen flüssigkeitsundurchlässigen Membran oder dadurch erfolgen, dass die Lösung vor deren Überführung ins zweite Reaktionsgefäß R2 auf Umgebungsdruck entspannt wird oder an die Lösung vor deren Überführung ins zweite Reaktionsgefäß R2 ein Unterdruck angelegt wird. Zur Erhöhung der Sicherheit kann außerdem das in der dritten Stufe des Verfahrens kondensierte Methanol entgast werden, um in der dritten Stufe des Verfahrens nicht umgesetztes und im Methanol gelöstes H₂ daraus zu entfernen. Das entfernte H₂ kann dem dritten Reaktionsgefäß R3 für die Hydrierung des Methylformiats zugeführt werden. Das Entgasen des Methanols kann dabei beispielsweise mittels einer Membran, insbesondere einer gasdurchlässigen flüssigkeitsundurchlässigen Membran, z. B. einer selektiven Holfasermembran, oder einer elektrochemischen Membran, wie bei dem elektrochemischen Wasserstoff-Separierungsverfahren EHS der Firma Siqens GmbH, München, oder mittels Druckwechseladsorption erfolgen.

Die Erfindung betrifft weiterhin eine Vorrichtung zur Durchführung des erfindungsgemäßen Verfahrens. Die Vorrichtung umfasst
- eine Elektrolyseeinheit E1,
- eine Oxidationseinheit mit einem ersten Reaktionsgefäß R1 und einer darin enthaltenen wässrigen Lösung des ersten Katalysators, wie er für das erfindungsgemäße Verfahren spezifiziert ist,
- eine Reaktivdestillationseinheit mit einem als Rektifikationskolonne ausgebildeten zweiten Reaktionsgefäß R2, in dem optional ein in flüssiger Form als Säure oder in fester Form als Schüttung vorliegender saurer zweiter Katalysator enthalten ist,
- einen Tank T zur Aufnahme von Methylformiat,
- einen Verdampfer V,
- eine Hydrierungseinheit mit einem dritten Reaktionsgefäß R3, in dem ein eine Hydrierung katalysierender dritter Katalysator enthalten ist,
- eine Kühl- und Kondensationseinheit K,
- einen weiteren Tank (Tw) und
- eine Wasserabscheideeinheit D1.

Dabei sind eine erste Gasleitung 7 zur Überführung von O₂ von der Elektrolyseeinheit zum ersten Reaktionsgefäß R1, eine zweite Gasleitung 21 zur Überführung von H₂ von der Elektrolyseeinheit zum dritten Reaktionsgefäß R3, eine erste Flüssigkeitsleitung 13 zur Überführung von Reaktionsflüssigkeit vom ersten Reaktionsgefäß R1 in das zweite Reaktionsgefäß R2, eine zweite Flüssigkeitsleitung 27 zur Überführung von Reaktionsflüssigkeit vom zweiten Reaktionsgefäß R2 in die Wasserabscheideeinheit D1, eine dritte Flüssigkeitsleitung 29 zur Überführung von Reaktionsflüssigkeit von der Wasserabscheideeinheit D1 in das erste Reaktionsgefäß R1, eine vierte Flüssigkeitsleitung 5 zur Überführung von Wasser von der Wasserabscheideeinheit D1 in die Elektrolyseeinheit E1, eine fünfte Flüssigkeitsleitung 15 zur Überführung von Methylformiat vom zweiten Reaktionsgefäß R2 in den Tank T, eine achte Flüssigkeitsleitung 17 zur Überführung von Methylformiat vom Tank T in den Verdampfer V, eine vierte Gasleitung 19 zur Überführung von verdampftem Methylformiat in das dritte Reaktionsgefäß R3, eine fünfte Gasleitung 22 zur Überführung von dampfförmigem Methanol aus dem dritten Reaktionsgefäß R3 in die Kühl- und Kondensationseinheit K, eine sechste Flüssigkeitsleitung 23 zur Überführung von in der Kühl- und Kondensationseinheit K verflüssigtem Methanol aus der Kühl- und Kondensationseinheit K in den weiteren Tank Tw, eine neunte Flüssigkeitsleitung 24 zur Ausleitung des Methanols aus dem weiteren Tank Tw, eine siebte Flüssigkeitsleitung 25 zur Überführung von Methanol aus der Kühl- und Kondensationseinheit K, der sechsten Flüssigkeitsleitung 23, dem weiteren Tank Tw oder der neunten Flüssigkeitsleitung 24 in das zweite Reaktionsgefäß R2 oder in die erste Flüssigkeitsleitung 13 und eine Regeleinheit vorgesehen. Die Regeleinheit steuert,
- dass ein für die Umsetzung sämtlicher erzeugter und in des zweite Reaktionsgefäß R2 überführter Ameisensäure zu Methylformiat erforderlicher konstanter Volumenstrom an Methanol über die siebte Flüssigkeitsleitung 25 in das zweite Reaktionsgefäß R2 geleitet wird und in der Elektrolyseeinheit E1 stets zumindest eine solche erste Menge an H₂ pro Minute erzeugt und dem dritten Reaktionsgefäß R3 über die zweite Gasleitung 21 zugeführt wird, dass genug Methanol erzeugt wird, um den konstanten Volumenstrom an Methanol aufrecht erhalten zu können, wobei vom Tank T stets eine solche Menge an Methylformiat in den Verdampfer V und von dort in das dritte Reaktionsgefäß R3 geleitet wird, dass sämtliches in das dritte Reaktionsgefäß R3 überführtes H₂ für eine Hydrierung des Methylformiats verbraucht wird,
- dass das im dritten Reaktionsgefäß R3 erzeugte Methanol mit dem konstanten Volumenstrom vollständig über die siebte Flüssigkeitsleitung 25 in das zweite Reaktionsgefäß R2 geleitet wird, wenn das Verfahren in einem ersten Verfahrensmodus durchgeführt wird und
- dass in der Elektrolyseeinheit E1 zusätzlich eine zweite Menge an H₂ pro Minute erzeugt und dem dritten Reaktionsgefäß R3 über die zweite Gasleitung 21 zusätzlich zur ersten Menge an H₂ pro Minute zugeführt wird und ein daraus resultierender nicht für die Aufrechterhaltung des konstanten Volumenstroms erforderlicher Anteil des erzeugten Methanols über die neunte Flüssigkeitsleitung 24 aus dem Verfahren ausgeschleust wird, wenn das Verfahren in einem zweiten Verfahrensmodus durchgeführt wird.

In der wässrigen Lösung des ersten Katalysators liegt der Katalysator in molekularer Form homogen in der Lösung verteilt vor. Die Vorrichtung kann so ausgebildet sein, dass in den genannten Leitungen lediglich Ventile zur Steuerung des Verfahrens vorgesehen sind, jedoch keine Verdichter und/oder Pumpen, wenn die Vorrichtung so aufgebaut ist, dass das jeweilige Gas aufgrund eines, beispielsweise bei der Elektrolyse aufgebauten Gasdrucks durch die jeweilige Gasleitung gedrückt wird oder die Lösung in dem jeweiligen Reaktionsgefäß aufgrund von hydrostatischem Druck durch die jeweilige Flüssigkeitsleitung bewegt oder aufgrund eines in dem jeweiligen Reaktionsgefäß über der Flüssigkeit bzw. Lösung aufgebauten Gasdrucks durch die jeweilige Flüssigkeitsleitung gedrückt wird. Es ist jedoch auch möglich, dass in der ersten Gasleitung 7 und/oder der zweiten Gasleitung 21 und/oder der vierten Gasleitung 19 und/oder der fünften Gasleitung 22 jeweils ein Verdichter zur Förderung und Erhöhung eines Drucks jeweils darin enthaltenen Gases/Dampfes vorgesehen ist und/oder dass zumindest in der ersten 13, zweiten 27, dritten 29, vierten 5, fünften 15, sechsten 23, siebten 25, achten 17 und/oder neunten Flüssigkeitsleitung 24 jeweils eine Pumpe zur Förderung jeweils darin enthaltenen Mediums vorgesehen ist.

Bei einer Ausgestaltung der erfindungsgemäßen Vorrichtung ist im ersten Reaktionsgefäß R1 eine Begasungseinheit, insbesondere ein statischer Mischer oder ein Rührwerk mit einer Gaseintragsvorrichtung oder in Kombination mit einer Gaseintragsvorrichtung, wie z. B. einer Begasungsleitung oder einem Begasungsring, zur Begasung des bei einer katalytischen Reaktion reduzierten ersten Katalysators mit O₂ vorgesehen.

Bei einer weiteren Ausgestaltung ist ein Druckwechseladsorptionsgerät zur Anreicherung von O₂ in oder Absonderung von O₂ aus Luft vorgesehen, wobei eine dritte Gasleitung vorgesehen ist, welche das Druckwechseladsorptionsgerät mit der ersten Gasleitung oder dem ersten Reaktionsgefäß R1 verbindet, um die O₂-angereicherte Luft oder das O₂ in die erste Gasleitung oder das erste Reaktionsgefäß R1 einzuspeisen.

Weiterhin kann bei der erfindungsgemäßen Vorrichtung im ersten Reaktionsgefäß R1 ein erster Wärmetauscher vorgesehen sein, um der darin enthaltenen Lösung in der zweiten Stufe des erfindungsgemäßen Verfahrens entstehende Wärme zu entziehen und abzuführen. Alternativ oder zusätzlich kann im dritten Reaktionsgefäß R3 ein zweiter Wärmetauscher vorgesehen sein, um in der dritten Stufe des erfindungsgemäßen Verfahrens entstehende Wärme zu entziehen und abzuführen.

Alternativ oder zusätzlich kann im zweiten Reaktionsgefäß R2 ein dritter Wärmetauscher oder eine erste Heizung vorgesehen sein, um der Reaktivdestillation in der zweiten Stufe des erfindungsgemäßen Verfahrens, insbesondere mittels des ersten und/oder zweiten Wärmetauschers abgeführte, Wärme zuzuführen.

Alternativ oder zusätzlich kann in der Wasserabscheideeinheit ein vierter Wärmetauscher oder eine zweite Heizung vorgesehen sein, um der Wasserabscheideeinheit, sofern erforderlich, insbesondere mittels des ersten und/oder zweiten Wärmetauschers abgeführte, Wärme zuzuführen.

Bei einer Ausgestaltung der erfindungsgemäßen Vorrichtung ist im ersten Reaktionsgefäß R1 mindestens ein erster Sensor, insbesondere ein erster pH-Sensor, ein erstes Refraktometer, ein erster Viskositätssensor, ein erster Dichtesensor, ein erster Inline-NMR-Sensor und/oder ein erstes Thermometer, zur Bestimmung einer Eigenschaft, insbesondere eines pH-Werts, eines Brechungsindex, einer Viskosität, einer Dichte, einer Zusammensetzung oder einer Temperatur, von Reaktionsflüssigkeit im ersten Reaktionsgefäß vorgesehen.

Alternativ oder zusätzlich kann im zweiten Reaktionsgefäß R2 mindestens ein zweiter Sensor, insbesondere ein zweiter pH-Sensor, ein zweites Refraktometer, ein zweiter Viskositätssensor, ein zweiter Dichtesensor, ein zweiter Inline-NMR-Sensor und/oder zweites Thermometer zur Bestimmung einer Eigenschaft, insbesondere eines pH-Werts, eines Brechungsindex, einer Viskosität, einer Dichte, einer Zusammensetzung oder einer Temperatur, von Reaktionsflüssigkeit im zweiten Reaktionsgefäß vorgesehen sein. Durch den ersten pH-Sensor lässt sich indirekt der Ameisensäuregehalt bei der Bildung der Ameisensäure im ersten Reaktionsgefäß R1 überwachen, da durch die Bildung der Ameisensäure der pH-Wert in der Lösung im ersten Reaktionsgefäß R1 sinkt. Durch den zweiten pH-Sensor lässt sich indirekt die Umsetzung der Ameisensäure zu Methylformiat im zweiten Reaktionsgefäß R2 überwachen, da bei der Umsetzung der pH-Wert steigt. Durch das erste Refraktometer lässt sich beispielsweise der Gehalt an noch nicht umgesetzten Glycerin im ersten Reaktionsgefäß R1 bestimmen. Durch den ersten Viskositätssensor und/oder den ersten Dichtesensor lässt sich der Gehalt an Ameisensäure bestimmen, weil die Viskosität und die Dichte der Reaktionsflüssigkeit im ersten Reaktionsgefäß R1 jeweils mit zunehmendem Ameisensäuregehalt abnimmt. Mittels des ersten und zweiten Inline-NMR-Sensors kann man jeweils ein genaues Bild der Zusammensetzung der Reaktionsflüssigkeit im ersten Reaktionsgefäß R1 und/oder zweiten Reaktionsgefäß R2 erhalten.

Alle in der Beschreibung angegebenen Merkmale sind im Sinne der Erfindung als Merkmale zu verstehen, die für alle Ausführungsformen der Erfindung gelten. Dies bedeutet beispielsweise, dass ein für das erfindungsgemäße Verfahren angegebenes Merkmal auch auf die erfindungsgemäße Vorrichtung angewendet werden kann und umgekehrt.

Nachfolgend wird die Erfindung anhand eines Ausführungsbeispiels näher erläutert.

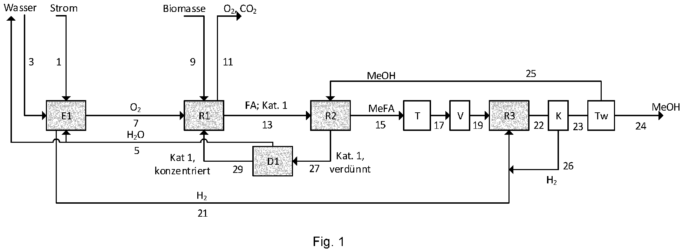

- Fig. 1: zeigt eine schematische Darstellung einer Vorrichtung zur Durchführung des erfindungsgemäßen Verfahrens.

In der Elektrolyseeinheit E1 wird mittels Strom, der über die Leitung 1 zugeführt wird, Wasser mittels Elektrolyse in Sauerstoff und Wasserstoff zerlegt. Am Beginn und in der Anfangsphase des Verfahrens muss dieses Wasser von außen über die Leitung 3 zugeführt werden. Später kann es aus dem Verfahren selbst gewonnen und über die vierte Flüssigkeitsleitung 5 der Elektrolyseeinheit E1 zugeführt werden. Der bei der Elektrolyse gebildete Sauerstoff wird einer im 1. Reaktionsgefäß R1 enthaltenen Begasungseinheit über die 1. Gasleitung 7 zugeführt.

Im ersten Reaktionsgefäß R1 ist eine wässrige Lösung des ersten Katalysators enthalten. Der erste Katalysator (Kat. 1) ist dabei H₈[PV₅Mo₇O₄₀] (HPA-5). Von außen wird dem ersten Reaktionsgefäß R1 Glycerin als Biomasse ("Biomasse" in Fig. 1) über die Leitung 9 zugeführt. Das erste Reaktionsgefäß R1 wird auf eine Temperatur im Bereich von 100 °C bis 120 °C, erwärmt. Im 1. Reaktionsgefäß R1 wird der darin gelöste Katalysator mittels der Begasungseinheit in Form eines eine Gaseintragsvorrichtung umfassenden Rührwerks begast. Dadurch wird der bei der Umsetzung der Biomasse zu Ameisensäure (FA) reduzierte Katalysator wieder in seinen oxidierten Ausgangszustand versetzt. Überschüssiger Sauerstoff und als Nebenprodukte gebildetes CO und CO₂ werden über die Leitung 11 und eine in Fig. 1 nicht dargestellte Kondensationsvorrichtung aus dem 1. Reaktionsgefäß R1 ausgeleitet. Die Kondensationsvorrichtung dient dabei dazu, den Teil der flüssigen Phase aus dem ersten Reaktionsgefäß R1, der das erste Reaktionsgefäß R1 dampfförmig über die Leitung 11 verlässt, zu kondensieren, um ihn dann wieder über die Leitung 11 oder eine weitere, hier nicht dargestellte Flüssigkeitsleitung in das erste Reaktionsgefäß R1 zurückzuführen.

Zunächst wird die Biomasse im ersten Reaktionsgefäß R1 umgesetzt, bis so viel Ameisensäure im ersten Reaktionsgefäß R1 produziert worden ist, dass deren Gehalt - nur bezogen auf die gebildete Ameisensäure und das Wasser im ersten Reaktionsgefäß R1, d. h. ohne Beachtung des Katalysators, der Biomasse und deren Abbauprodukte und andere Reaktionsprodukte als Ameisensäure - im Bereich von 25 - 65 Gew.-%, insbesondere 40 - 60 Gew.-% liegt. Je nach Art der Biomasse dauert das zwischen 2 und 12 Stunden. Der Gehalt an Ameisensäure wird dabei und im folgenden Verfahren mittels eines Inline-NMR-Sensors über Online-NMR überwacht. Danach wird kontinuierlich pro Stunde 1/10 der Lösung im ersten Reaktionsgefäß R1, die den Katalysator, gebildete Ameisensäure, nicht umgesetzte Biomasse, deren Abbauprodukte und andere Reaktionsprodukte als Ameisensäure enthält, über die erste Flüssigkeitsleitung 13 aus dem ersten Reaktionsgefäß R1 ausgeleitet und in das zweite Reaktionsgefäß R2 eingeleitet. Aus der Ausleitung von 1/10 der im ersten Reaktionsgefäß R1 befindlichen Lösung pro Stunde resultiert eine hydrodynamische Verweilzeit von 10 Stunden im ersten Reaktionsgefäß R1. Je nach Art der Biomasse und gewählten Reaktionsbedingungen kann auch eine deutlich kürzere oder längere hydrodynamische Verweilzeit gewählt werden. Als günstig hat sich eine hydrodynamische Verweilzeit im Bereich von 1 bis 20 Stunden, insbesondere 2 bis 10 Stunden, erwiesen. Weiterhin wird dem ersten Reaktionsgefäß kontinuierlich Biomasse zugeführt.

In das zweite Reaktionsgefäß R2 wird über die siebte Flüssigkeitsleitung 25 Methanol (MeOH) eingeleitet. Das zweite Reaktionsgefäß ist als Rektifikationskolonne ausgebildet und enthält einen sauren in fester Form vorliegenden zweiten Katalysator als Schüttung. Im vorliegenden Fall handelt es sich beim zweiten Katalysator um Amberlyst^{®} 15H. Amberlyst^{®} 15H liegt dabei gemischt mit Raschigringen im Gewichtsverhältnis 2:1 vor. Die Rektifikationskolonne wird auf eine Temperatur von 98 °C im Sumpf erhitzt. Dadurch wird eine Reaktivdestillation in Gang gesetzt und durch Veresterung von Ameisensäure mit Methanol Methylformiat (MeFA) gebildet. Das entstandene Methylformiat wird aus der Rektifikationskolonne mit einem Rücklaufverhältnis von 4:1 ausgeschleust. Dabei können eine Reinheit von über 99% und ein Wasseranteil unter 400 ppm erreicht werden. Das ausgeschleuste Methylformiat wird über die fünfte Flüssigkeitsleitung 15 in den Tank T und von diesem über eine achte Flüssigkeitsleitung 17 mittels einer hier nicht dargestellten Pumpe P in einen Verdampfer V gepumpt. Darin entstehendes gasförmiges Methylformiat wird über eine vierte Gasleitung 19 und einem hier nicht dargestellten Verdichter dem dritten Reaktionsgefäß R3 zugeführt. Weiterhin wird über die zweite Gasleitung 21 Wasserstoff aus der Elektrolyseeinheit E1 in das dritte Reaktionsgefäß R3 eingeleitet und mit dem gasförmigen Methylformiat nach Möglichkeit in einem Molverhältnis von Wasserstoff zu Methylformiat von 5,8:1 vermischt. Das dritte Reaktionsgefäß R3 ist als Rohrbündelreaktor ausgeführt und mit dem eine Hydrierung katalysierenden dritter Katalysator gefüllt. Dabei handelt es sich im vorliegenden Fall um einen Cu_{0.9}Al₂O₄ Spinel-Typ-Katalysator. Im dritten Reaktionsgefäß R3 sind ein Druck im Bereich von 8 bis 12 bar und eine Temperatur im Bereich von 150 °C bis 260 °C eingestellt. Dabei erfolgt eine Hydrierung und eine Hydrogenolyse des Methylformiats zu dampfförmigem Methanol. Das gebildete dampfförmige Methanol wird über eine fünfte Gasleitung 22 einer Kühl- und Kondensationseinheit K zugeführt. Darin bildet sich durch Kühlung und Kondensation flüssiges Methanol. Gegebenenfalls bei der Hydrierung nicht verbrauchter Wasserstoff bleibt in der Gasphase zurück und kann aus der Kühl- und Kondensationseinheit ausgeleitet oder optional, beispielsweise mittels eines Membranverfahrens oder Druckwechseladsorption, separiert und gegebenenfalls nach Kompression mittels eines weiteren hier nicht dargestellten Verdichters über eine sechste Gasleitung 26 wieder dem Reaktionsgefäß R3 - direkt oder indirekt durch Einleitung in die zweite Gasleitung 21 - für die Hydrierung des Methylformiats zugeführt werden. Das flüssige Methanol wird durch eine sechste Flüssigkeitsleitung 23 aus der Kühl- und Kondensationseinheit K ausgeleitet. Die sechsten Flüssigkeitsleitung 23 mündet in einen weiteren Tank Tw, von dem eine siebte Flüssigkeitsleitung 25 abzweigt. Alternativ kann die siebte Flüssigkeitsleitung 25 auch von der sechsten Flüssigkeitsleitung 23 oder direkt von der Kühl- und Kondensationseinheit K abzweigen. Die siebte Flüssigkeitsleitung 25 mündet in das zweite Reaktionsgefäß R2 und dient dazu, Methanol in das zweite Reaktionsgefäß R2 einzuleiten. Dazu wird von dem weiteren Tank Tw mittels einer nicht dargestellten Pumpe ein konstanter Volumenstrom an Methanol in die siebte Flüssigkeitsleitung 25 geleitet. In dem weiteren Tank Tw ist außerdem ein Überlauf vorgesehen, von dem aus nicht in die siebte Flüssigkeitsleitung 25 geleitetes Methanol über eine neunte Flüssigkeitsleitung 24 ausgeleitet wird. Wird das Verfahren im ersten Verfahrensmodus betrieben, werden 100 % des erzeugten Methanols in die siebte Flüssigkeitsleitung 25 geleitet. Wird das Verfahren im zweiten Verfahrensmodus betrieben, wird nur ein Teil des erzeugten Methanols in die siebte Flüssigkeitsleitung 25 geleitet und der Rest des erzeugten Methanols wird über die neunte Flüssigkeitsleitung 24 ausgeleitet. Der Volumenstrom des durch die siebte Flüssigkeitsleitung 25 dem zweiten Reaktionsgefäß R2 zugeführten Methanols ist aber im ersten Verfahrensmodus und im zweiten Verfahrensmodus identisch.

Über eine in Fig. 1 nicht dargestellte Regeleinheit wird in Abhängigkeit von einer in einem System zur Verfügung stehenden Strommenge im Verhältnis zu dem in dem System bestehenden Strombedarf geregelt, wieviel H₂ erzeugt und dem dritten Reaktionsgefäß R3 zugeführt wird und wieviel Methylformiat dem Verdampfer V zur anschließenden Hydrierung im dritten Reaktionsgefäß R3 zugeführt wird. Ist das Verhältnis "zur Verfügung stehende Strommenge : Strombedarf" kleiner oder gleich 1, wird das Verfahren vorzugsweise im ersten Verfahrensmodus betrieben, anderenfalls, d. h. wenn das Verhältnis größer als 1 ist, im zweiten Verfahrensmodus. Wenn das genannte Verhältnis kleiner oder gleich 1 ist, wird das Verfahren durch die Regeleinheit beispielsweise so geregelt, dass nur so viel H₂ erzeugt wird und nur so viel Methylformiat dem Verdampfer V zur anschließenden Hydrierung im dritten Reaktionsgefäß R3 zugeführt wird, wie es zur Erzeugung der Methanolmenge zur Aufrechterhaltung des konstanten Volumenstroms an Methanol in der siebten Flüssigkeitsleitung 25 erforderlich ist. Das im dritten Reaktionsgefäß R3 erzeugte Methanol wird dann vollständig über die siebte Flüssigkeitsleitung 25 in das zweite Reaktionsgefäß R2 geleitet. Wenn das Verhältnis größer als 1 ist, wird das Verfahren durch die Regeleinheit so geregelt, dass mehr H₂ erzeugt und mehr Methylformiat dem Verdampfer V zur anschließenden Hydrierung im dritten Reaktionsgefäß R3 zugeführt wird. Von dem im dritten Reaktionsgefäß R3 erzeugten Methanol wird dann mit dem konstanten Volumenstrom nur der Anteil, der erforderlich ist, um sämtliche Ameisensäure im zweiten Reaktionsgefäß damit zu Methylformiat umzusetzen, vom weiteren Tank Tw über die siebte Flüssigkeitsleitung 25 in das zweite Reaktionsgefäß R2 geleitet und ein darüber hinaus gehender Anteil des erzeugten Methanols über die neunte Flüssigkeitsleitung 24 aus dem Verfahren ausgeschleust. Das genannte Verhältnis spiegelt sich üblicherweise im Strompreis der Stromanbieter wider. Je mehr das Verhältnis 1 übersteigt, desto günstiger ist der Strom und je weiter das Verhältnis unterhalb von 1 liegt, desto teurer ist der Strom üblicherweise.

Der bei der Reaktivdestillation im zweiten Reaktionsgefäß R2 verbleibende Rückstand, das sogenannte Sumpfprodukt, enthält unter anderem den verdünnten ersten Katalysator und gegebenenfalls nicht umgesetzte Biomasse.

Um den im Sumpfprodukt enthaltenen Katalysator bei Bedarf zu konzentrieren, damit er katalytisch wieder eine größere Wirksamkeit entfaltet, wird das Sumpfprodukt aus dem zweiten Reaktionsgefäß R2 über die zweite Flüssigkeitsleitung 27 in die Wasserabscheideeinheit D1 übergeleitet, die hier als Entspannungsverdampfer ausgebildet ist. Das dadurch konzentrierte Sumpfprodukt wird über die dritte Flüssigkeitsleitung 29 in das erste Reaktionsgefäß R1 eingeleitet. Hier wird darin gegebenenfalls noch enthaltene Biomasse und neu hinzugefügte Biomasse wie oben bereits beschrieben mittels des Katalysators zu Ameisensäure umgesetzt. In der Wasserabscheideeinheit D1 abgeschiedenes Wasser wird über die vierte Flüssigkeitsleitung 5 der Elektrolyseeinheit E1 zugeführt und dort elektrolytisch in Sauerstoff und Wasserstoff zerlegt.

In Fig. 1 nicht dargestellt sind jeweils ein Wärmetauscher im ersten Reaktionsgefäß R1 und dritten Reaktionsgefäß R3, um die darin jeweils frei werdende Wärme abzuführen. Weiterhin ist im ersten Reaktionsgefäß R1 eine in Fig. 1 nicht dargestellte Heizung enthalten, die lediglich dazu dient, die darin erfolgende und eigentlich Wärme freisetzende Oxidationsreaktion in Gang zu setzen.

Weiterhin nicht dargestellt sind ein Wärmetauscher und eine Heizung im zweiten Reaktionsgefäß R2, die beide dazu dienen, die darin enthaltene Rektifikationskolonne zu beheizen. Am Anfang dient dazu die, beispielsweise elektrisch betriebene, Heizung. Sobald im ersten Reaktionsgefäß R1 und im dritten Reaktionsgefäß R3 genug Wärme freigesetzt wird, wird diese über den Wärmetauscher der Rektifikationskolonne im zweiten Reaktionsgefäß zugeführt. Ein zusätzliches Beheizen der Rektifikationskolonne über die Heizung ist dann nicht mehr erforderlich.

Weiterhin nicht dargestellt sind ein Wärmetauscher und eine Heizung in der Wasserabscheideeinheit D1, die beide dazu dienen, den Entspannungsverdampfer zu beheizen. Am Anfang dient dazu die, insbesondere elektrisch betriebene, Heizung. Sobald im ersten Reaktionsgefäß R1 und im dritten Reaktionsgefäß R3 genug Wärme freigesetzt wird, wird diese über den Wärmetauscher der Wasserabscheideeinheit D1 zugeführt. Ein zusätzliches Beheizen der Wasserabscheideeinheit D1 über die Heizung ist dann nicht mehr oder nur in geringem Ausmaß erforderlich.

Ebenfalls in Fig. 1 nicht dargestellt sind Verdichter zur Förderung von Gas durch die jeweiligen Gasleitungen und zum Druckaufbau und Pumpen zur Förderung von Flüssigkeit durch die jeweiligen Flüssigkeitsleitungen, soweit erforderlich.

In einem Ausführungsbeispiel wird das Verfahren zu 75% eines Tages im zweiten Verfahrensmodus betrieben, so dass Methanol ausgeschleust wird und zu 25% eines Tages im ersten Verfahrensmodus betrieben, so dass nur Methylformiat erzeugt und im Tank T gespeichert wird. Dazu sind die Elektrolyseeinheit E1 bezüglich ihrer Leistungsfähigkeit und das erste Reaktionsgefäß R1 bezüglich seiner Größe so ausgelegt, dass die in den 25% des Tages erzeugte und im Tank T gespeicherte Menge an Methylformiat in den restlichen 75% des Tages zu Methanol (MeOH) umgesetzt werden kann. Das bedeutet, dass in den 75 % des Tages genug Wasserstoff erzeugt wird und das dritte Reaktionsgefäß R3 hinsichtlich der Größe so ausgelegt ist, dass die aus dieser Größe resultierende Verweilzeit ausreicht, um das im zweiten Verfahrensmodus kontinuierlich erzeugte und das im Tank gespeicherte Methylformiat (MeFA) innerhalb dieser 75% eines Tages zu hydrieren. Der daraus resultierende Verweilzeitfaktor, d. h. der Faktor, um den sich die Verweilzeit im dritten Reaktionsgefäß R3 im ersten Verfahrensmodus durch den geringeren Volumenstrom gegenüber der entsprechenden Verweilzeit im zweiten Verfahrensmodus verlängert, und die Auslastung der Elektrolyseeinheit E1 im Verhältnis zur maximalen Auslastung der Elektrolyseeinheit E1 ergeben sich aus der folgenden Tabelle 1:

**Tabelle 1:**

| | Betriebszeit [h/d] | Verweilzeitfaktor für R3 [-] | Auslastung E1 bezogen auf maximale H2 Erzeugung[-] |
|---|---|---|---|
| Erster Verfahrensmodus | 6 | 2,33 | 44% |
| Zweiter Verfahrensmodus | 18 | 1,00 | 100% |

In diesem Ausführungsbeispiel ergibt sich aus dem sechststündigen Betrieb im ersten Verfahrensmodus, d. h. mit reduziertem Strombedarf, ein zur Speicherung von Methylformiat im Tank T nötiges spezifisches Volumen des Tanks T von 0,24 L pro kWh Stromverbrauch im ersten Verfahrensmodus.

In einem weiteren Ausführungsbeispiel wird das Verfahren zu 25% eines Tages im zweiten Verfahrensmodus betrieben, so dass Methanol ausgeschleust wird und zu 75% eines Tages im ersten Verfahrensmodus betrieben, so dass nur Methylformiat erzeugt und im Tank T gespeichert wird. Das bedeutet, dass die in 75% der Zeit erzeugte und im Tank T gespeicherte Menge an Methylformiat in 25% der Zeit mit Wasserstoff zu Methanol umgesetzt wird. Dazu ist die Elektrolyseeinheit E1 bezüglich ihrer Leistungsfähigkeit so ausgelegt, dass dafür genügend Wasserstoff während der 25% eines Tages erzeugt werden kann. Das erste Reaktionsgefäß R1 ist bezüglich seiner Größe so ausgelegt, dass die aus dieser Größe resultierende Verweilzeit ausreicht, um das im zweiten Verfahrensmodus kontinuierlich erzeugte und das im Tank gespeicherte Methylformiat (MeFA) innerhalb dieser 25% eines Tages zu hydrieren. Aus den unterschiedlichen Zeiten im ersten und im zweiten Verfahrensmodus ergibt sich, dass die Auslastung der Elektrolyseeinheit E1 im Verhältnis zur maximalen Auslastung der Elektrolyseeinheit E1 und die Verweilzeit im dritten Reaktionsgefäß R3 während der bloßen Erzeugung von Methylformiat, d. h. im ersten Verfahrensmodus, gegenüber dem ersten Ausführungsbeispiel reduziert sind. Dies ist in der folgenden Tabelle 2 verdeutlicht:

**Tabelle 2:**

| | Betriebszeit [h/d] | Verweilzeitfaktor für R3 [-] | Auslastung E1 bezogen auf maximale H2 Erzeugung[-] |
|---|---|---|---|
| Erster Verfahrensmodus | 18 | 5 | 20% |
| Zweiter Verfahrensmodus | 6 | 1,00 | 100% |

In diesem Ausführungsbeispiel ergibt sich aus dem achtzehnstündigen Betrieb im ersten Verfahrensmodus, d. h. mit reduziertem Strombedarf, ein zur Speicherung von Methylformiat im Tank T nötiges spezifisches Volumen des Tanks T von 0,08 L pro kWh Stromverbrauch im ersten Verfahrensmodus. Das zur Pufferung nötige Tankvolumen ist in diesem Fall kleiner als im vorherigen Ausführungsbeispiel, da der Unterschied zwischen maximaler und minimaler Auslastung der Elektrolyseeinheit E1 größer ist als im vorherigen Ausführungsbeispiel.

## Patentansprüche

1. Verfahren zur katalytischen Erzeugung von Methanol aus Biomasse mittels elektrischem Strom, wobei die Biomasse ein Material ist, welches mindestens ein alpha-Hydroxyaldehyd, mindestens eine alpha-Hydroxycarbonsäure, mindestens ein Kohlenhydrat und/oder mindestens ein Glycosid umfasst oder daraus besteht,
- wobei in einer ersten Stufe aus Wasser durch Elektrolyse mittels des Stroms O₂ und H₂ erzeugt wird,
- wobei die Biomasse in einer zweiten Stufe mittels einer wässrigen Lösung eines ersten Katalysators in einem ersten Reaktionsgefäß (R1) zu Ameisensäure umgesetzt wird, wobei der bei der katalytischen Reaktion reduzierte erste Katalysator durch Oxidation wieder in seinen Ausgangszustand versetzt wird, wobei zur Oxidation des reduzierten Katalysators das in der ersten Stufe erzeugte O₂ in die Lösung im ersten Reaktionsgefäß (R1) eingebracht wird, wobei der erste Katalysator ein Polyoxometallat-Ion der allgemeinen Formel [PMoₓV_{y}O₄₀]ⁿ⁻ ist, wobei 6 ≤ x ≤ 11, 1 ≤ y ≤ 6 und x + y = 12, [W×V_{y}O₁₉]ⁿ⁻ ist, wobei x + y = 6, 3 ≤ x ≤ 5 und 1 ≤ y ≤ 3, oder [P₂WₓV_{y}O₆₂]ⁿ⁻ ist, wobei x + y = 18, 12 ≤ x ≤ 17 und 1 ≤ y ≤ 6, oder ein VO²⁺ enthaltendes Salz oder ein [VO₃]⁻ enthaltendes Salz ist, wobei n, x und y jeweils eine ganze Zahl ist, wobei die Lösung mit der darin entstandenen Ameisensäure in ein zweites Reaktionsgefäß (R2) überführt wird, wobei der Lösung beim Überführen in das zweite Reaktionsgefäß oder im zweiten Reaktionsgefäß (R2) Methanol zugesetzt wird, wobei das zweite Reaktionsgefäß (R2) als Rektifikationskolonne ausgebildet ist, in der optional ein eine Veresterung des Methanols mit der Ameisensäure katalysierender saurer zweiter Katalysator enthalten ist, wobei der zweite Katalysator in fester Form als Schüttung oder in flüssiger Form als Säure vorliegt, wobei im zweiten Reaktionsgefäß (R2) eine Reaktivdestillation durchgeführt und das dabei entstehende Methylformiat in einen Tank (T) überführt wird,
- wobei in einer dritten Stufe Methylformiat aus dem Tank (T) verdampft und in ein drittes Reaktionsgefäß (R3) überführt und dort mit dem H₂ aus der ersten Stufe mittels eines eine Hydrierung katalysierenden dritten Katalysators hydriert wird, wobei durch Hydrogenolyse dampfförmiges Methanol entsteht, welches anschließend aus dem dritten Reaktionsgefäß (R3) ausgeleitet und so weit abgekühlt wird, dass das Methanol kondensiert,
- wobei in einer vierten Stufe der in der zweiten Stufe im zweiten Reaktionsgefäß (R2) verbleibende, den ersten Katalysator enthaltende Rückstand der Reaktivdestillation durch Wasserabscheidung konzentriert und dem ersten Reaktionsgefäß (R1) zugeführt wird,
wobei der Strom in einem ersten Verfahrensmodus zur Erzeugung von H₂ in der ersten Stufe eingesetzt wird, welches in der dritten Stufe mit dem Methylformiat zu Methanol umgesetzt wird, das vollständig in das zweite Reaktionsgefäß (R2) überführt und in der zweiten Stufe mit der Ameisensäure zu Methylformiat umgesetzt wird, wobei der Strom in einem zweiten Verfahrensmodus zur Erzeugung von H₂ in der ersten Stufe eingesetzt wird, welches in der dritten Stufe mit dem Methylformiat zu Methanol umgesetzt wird, das in das zweite Reaktionsgefäß (R2) überführt und in der zweiten Stufe mit der Ameisensäure zu Methylformiat umgesetzt wird und außerdem in der ersten Stufe zum Erzeugen von zusätzlichem H₂ eingesetzt wird, wobei dieses zusätzliche H₂ in der dritten Stufe zum Erzeugen von zusätzlichem Methanol eingesetzt wird, welches aus dem Verfahren ausgeschleust wird, wobei das Verfahren abwechselnd im ersten und im zweiten Verfahrensmodus betrieben wird.

2. Verfahren nach Anspruch 1, wobei das Methanol in der zweiten Stufe zumindest in einer solchen Menge zugesetzt wird, dass sämtliche Ameisensäure im zweiten Reaktionsgefäß damit zu Methylformiat umgesetzt wird.

3. Verfahren nach Anspruch 2, wobei am Beginn einer Durchführung des Verfahrens in der zweiten Stufe Methanol von außen zugeführt wird, wobei es nur so lange und nur in einer solchen Menge von außen zugeführt wird, wie es erforderlich ist, damit insgesamt in der zweiten Stufe zusammen mit dem aus der dritten Stufe stammenden und in das zweite Reaktionsgefäß (R2) überführten Methanol genug Methanol zur Verfügung steht, um damit sämtliche Ameisensäure im zweiten Reaktionsgefäß (R2) zu Methylformiat umzusetzen.

4. Verfahren nach einem der vorhergehenden Ansprüche, wobei mittels Druckwechseladsorption O₂ aus Luft separiert oder eine O₂-angereicherte Luft erzeugt wird und dieses O₂ oder diese O₂-angereicherte Luft zur Oxidation des bei der katalytischen Reaktion reduzierten ersten Katalysators zusätzlich zum in der ersten Stufe erzeugten O₂ in die Lösung im ersten Reaktionsgefäß (R1) eingebracht wird und/oder wobei zur Oxidation des bei der katalytischen Reaktion reduzierten ersten Katalysators zusätzlich zum in der ersten Stufe erzeugten O₂ Luft in die Lösung im ersten Reaktionsgefäß (R1) eingebracht wird.

5. Verfahren nach einem der vorhergehenden Ansprüche, wobei der zweite Katalysator in flüssiger Form Salzsäure oder Schwefelsäure und der zweite Katalysator in fester Form ZnO/ZrO₂ oder ein, insbesondere poröser, geträgerter, insbesondere Siliziumdioxid-geträgerter, Diarylammonium-Katalysator, ein saurer Kationenaustauscher, insbesondere ein saurer Zeolith oder ein Polystyrolsulfonat, eine geträgerte, insbesondere von Siliziumdioxid, Aktivkohle oder porösem Glas geträgerte, Heteropolysäure, insbesondere Wolframatophosphorsäure, 2-[1-[Difluor[(trifluorethenyl)oxy]methyl]-1,2,2,2-tetrafluorethoxy]-1,1,2,2-tetrafluorethan-sulfonsäure (Nafion^{®}) oder mesoporöses Silika, ist.

6. Verfahren nach einem der vorhergehenden Ansprüche, wobei der dritte Katalysator ein aus mindestens einem Metall, insbesondere Pt , Pd, Rh, Ru, Ni, Co, Fe und Cu, in elementarer oder oxidierter Form bestehender oder ein solches Metall enthaltender und in fester Form vorliegender Hydrierungskatalysator, insbesondere Platinschwarz, Platin(IV)-oxid, Palladiumschwarz, kolloides Palladium, Palladium(II)-oxid, Palladium auf Calciumcarbonat oder Bariumsulfat, ein Lindlar-Katalysator, ein Nishimura-Katalysator, Nickel auf Kieselgur, ein Raney-Nickel-Katalysator, Kupferchromit (CuCr₂O₄), ein Adkins-Katalysator, ein Kupfer-Zink, Kupfer-Alumina oder Kupfer-Bismuth Katalysator, Raney-Cobalt, ein Cu_{0.9}Al₂O₄ Spinel-Typ-Katalysator, ein CuO/Cr₂O₃-Katalysator, ein CuO/MgO/ZnO/Al₂O₃-Katalysator, ein Cu-ZnO₂-Katalysator, ein Cu-SiO₂-Katalysator, ein Raney-Kupfer Katalysator oder ein Kupfer-Aluminium-Legierung-Katalysator, ist.

7. Verfahren nach einem der vorhergehenden Ansprüche, wobei die Wasserabscheidung in der vierten Stufe durch Verdampfung, insbesondere eine Entspannungsverdampfung, oder durch Pervaporation erfolgt oder wobei die Wasserabscheidung mittels einer Rektifikation oder zweistufigen Destillation erfolgt, mittels der auch eine Abscheidung von in dem Rückstand noch enthaltenem Methanol erfolgt.

8. Verfahren nach einem der vorhergehenden Ansprüche, wobei im ersten Verfahrensmodus das in der ersten Stufe erzeugte H₂ teilweise durch H₂ ergänzt oder ersetzt wird, das durch Dehydrierung des in der zweiten Stufe erzeugten Methylformiats mittels eines Dehydrierungskatalysators erzeugt wird.

9. Verfahren nach einem der vorhergehenden Ansprüche, wobei in der vierten Stufe abgeschiedenes Wasser der Elektrolyse in der ersten Stufe zugeführt wird und optional hinsichtlich des pH-Werts durch Zusatz einer Base oder Säure, hinsichtlich des elektrischen Widerstands mittels Abscheiden des Wassers durch eine semipermeable Membran hindurch, insbesondere durch eine Umkehrosmose, und/oder hinsichtlich der Ionenkonzentration durch Hindurchleiten durch einen oder mehrere Ionenaustauscher an das Wasser in der ersten Stufe des Verfahrens angeglichen wird, bevor es der Elektrolyse in der ersten Stufe zugeführt wird.

10. Verfahren nach einem der vorhergehenden Ansprüche, wobei im ersten Reaktionsgefäß (R1) in der zweiten Stufe entstehende Wärme mittels eines ersten Wärmetauschers und im dritten Reaktionsgefäß (R3) in der dritten Stufe entstehende Wärme mittels eines zweiten Wärmetauschers abgeführt und dem zweiten Reaktionsgefäß (R2) mittels eines dritten Wärmetauschers und/oder der Wasserabscheideeinheit (D1), sofern erforderlich, mittels eines vierten Wärmetauschers zugeführt wird.

11. Vorrichtung zur Durchführung des Verfahrens nach einem der vorhergehenden Ansprüche, wobei die Vorrichtung
- eine Elektrolyseeinheit (E1),
- eine Oxidationseinheit mit einem ersten Reaktionsgefäß (R1) und einer darin enthaltenen wässrigen Lösung des ersten Katalysators, wie er in Patentanspruch 1 spezifiziert ist,
- eine Reaktivdestillationseinheit mit einem als Rektifikationskolonne ausgebildeten zweiten Reaktionsgefäß (R2), in dem optional ein in flüssiger Form als Säure oder in fester Form als Schüttung vorliegender saurer zweiter Katalysator enthalten ist,
- einen Tank (T) zur Aufnahme von Methylformiat,
- einen Verdampfer (V),
- eine Hydrierungseinheit mit einem dritten Reaktionsgefäß (R3), in dem ein eine Hydrierung katalysierender dritter Katalysator enthalten ist,
- eine Kühl- und Kondensationseinheit (K),
- einen weiteren Tank (Tw) und
- eine Wasserabscheideeinheit (D1) umfasst,
wobei eine erste Gasleitung (7) zur Überführung von O₂ von der Elektrolyseeinheit zum ersten Reaktionsgefäß (R1), eine zweite Gasleitung (21) zur Überführung von H₂ von der Elektrolyseeinheit zum dritten Reaktionsgefäß (R3), eine erste Flüssigkeitsleitung (13) zur Überführung von Reaktionsflüssigkeit vom ersten Reaktionsgefäß (R1) in das zweite Reaktionsgefäß (R2), eine zweite Flüssigkeitsleitung (27) zur Überführung von Reaktionsflüssigkeit vom zweiten Reaktionsgefäß (R2) in die Wasserabscheideeinheit (D1), eine dritte Flüssigkeitsleitung (29) zur Überführung von Reaktionsflüssigkeit von der Wasserabscheideeinheit (D1) in das erste Reaktionsgefäß (R1), eine vierte Flüssigkeitsleitung (5) zur Überführung von Wasser von der Wasserabscheideeinheit (D1) in die Elektrolyseeinheit (E1), eine fünfte Flüssigkeitsleitung (15) zur Überführung von Methylformiat vom zweiten Reaktionsgefäß (R2) in den Tank (T), eine achte Flüssigkeitsleitung (17) zur Überführung von Methylformiat vom Tank T in den Verdampfer (V), eine vierte Gasleitung (19) zur Überführung von verdampftem Methylformiat in das dritte Reaktionsgefäß (R3), eine fünfte Gasleitung (22) zur Überführung von dampfförmigem Methanol aus dem dritten Reaktionsgefäß (R3) in die Kühl- und Kondensationseinheit (K), eine sechste Flüssigkeitsleitung (23) zur Überführung von in der Kühl- und Kondensationseinheit K verflüssigtem Methanol aus der Kühl- und Kondensationseinheit (K) in den weiteren Tank (Tw), eine neunte Flüssigkeitsleitung (24) zur Ausleitung des Methanols aus dem weiteren Tank (Tw), eine siebte Flüssigkeitsleitung (25) zur Überführung von Methanol aus der Kühl- und Kondensationseinheit (K), der sechsten Flüssigkeitsleitung (23), dem weiteren Tank (Tw) oder der neunten Flüssigkeitsleitung (24) in das zweite Reaktionsgefäß (R2) oder in die erste Flüssigkeitsleitung (13) und eine Regeleinheit vorgesehen sind, wobei die Regeleinheit steuert,
- dass ein für die Umsetzung sämtlicher erzeugter und in des zweite Reaktionsgefäß (R2) überführter Ameisensäure zu Methylformiat erforderlicher konstanter Volumenstrom an Methanol über die siebte Flüssigkeitsleitung (25) in das zweite Reaktionsgefäß (R2) geleitet wird und in der Elektrolyseeinheit (E1) stets zumindest eine solche erste Menge an H₂ pro Minute erzeugt und dem dritten Reaktionsgefäß (R3) über die zweite Gasleitung (21) zugeführt wird, dass genug Methanol erzeugt wird, um den konstanten Volumenstrom an Methanol aufrecht erhalten zu können, wobei vom Tank (T) stets eine solche Menge an Methylformiat in den Verdampfer (V) und von dort in das dritte Reaktionsgefäß (R3) geleitet wird, dass sämtliches in das dritte Reaktionsgefäß (R3) überführtes H₂ für eine Hydrierung des Methylformiats verbraucht wird,
- dass das im dritten Reaktionsgefäß (R3) erzeugte Methanol mit dem konstanten Volumenstrom vollständig über die siebte Flüssigkeitsleitung (25) in das zweite Reaktionsgefäß (R2) geleitet wird, wenn das Verfahren in einem ersten Verfahrensmodus durchgeführt wird und
- dass in der Elektrolyseeinheit (E1) zusätzlich eine zweite Menge an H₂ pro Minute erzeugt und dem dritten Reaktionsgefäß (R3) über die zweite Gasleitung (21) zusätzlich zur ersten Menge an H₂ pro Minute zugeführt wird und ein daraus resultierender nicht für die Aufrechterhaltung des konstanten Volumenstroms erforderlicher Anteil des erzeugten Methanols über die neunte Flüssigkeitsleitung (24) aus dem Verfahren ausgeschleust wird, wenn das Verfahren in einem zweiten Verfahrensmodus durchgeführt wird.

12. Vorrichtung nach Anspruch 11, wobei in der ersten Gasleitung (7) und/oder der zweiten Gasleitung (21) und/oder der vierten Gasleitung (19) und/oder der fünften Gasleitung (22) jeweils ein Verdichter zur Förderung oder Erhöhung eines Drucks jeweils darin enthaltenen Gases/Dampfes vorgesehen ist und/oder wobei zumindest in der ersten (13), zweiten (27), dritten (29), vierten (5), fünften (15), sechsten (23), siebten (25), achten (17) und/oder neunten Flüssigkeitsleitung (24) jeweils eine Pumpe zur Förderung jeweils darin enthaltenen Mediums vorgesehen ist.

13. Vorrichtung nach Anspruch 11 oder 12, wobei im ersten Reaktionsgefäß (R1) eine Begasungseinheit, insbesondere ein statischer Mischer oder ein eine Gaseintragsvorrichtung umfassendes Rührwerk oder ein Rührwerk in Kombination mit einer Gaseintragsvorrichtung, zur Begasung des bei einer katalytischen Reaktion reduzierten ersten Katalysators mit O₂ vorgesehen ist und/oder wobei ein Druckwechseladsorptionsgerät zur Anreicherung von O₂ in oder Absonderung von O₂ aus Luft vorgesehen ist, wobei eine dritte Gasleitung vorgesehen ist, welche das Druckwechseladsorptionsgerät mit der ersten Gasleitung (7) oder dem ersten Reaktionsgefäß verbindet, um die O₂-angereicherte Luft oder das O₂ in die erste Gasleitung (7) oder das erste Reaktionsgefäß (R1) einzuspeisen.

14. Vorrichtung nach einem der Ansprüche 11 bis 13, wobei im ersten Reaktionsgefäß (R1) ein erster Wärmetauscher vorgesehen ist, um der darin enthaltenen Lösung in der zweiten Stufe des Verfahrens nach einem der Ansprüche 1 bis 10 entstehende Wärme zu entziehen und abzuführen und/oder im dritten Reaktionsgefäß (R3) ein zweiter Wärmetauscher vorgesehen ist, um in der dritten Stufe des Verfahrens nach einem der Ansprüche 1 bis 10 entstehende Wärme zu entziehen und abzuführen und/oder im zweiten Reaktionsgefäß (R2) ein dritter Wärmetauscher oder eine erste Heizung vorgesehen ist, um der Reaktivdestillation in der zweiten Stufe des Verfahrens nach einem der Ansprüche 1 bis 10, insbesondere mittels des ersten und/oder zweiten Wärmetauschers abgeführte, Wärme zuzuführen und/oder in der Wasserabscheideeinheit (D1) ein vierter Wärmetauscher oder eine zweite Heizung vorgesehen ist, um der Wasserabscheideeinheit (D1), sofern erforderlich, insbesondere mittels des ersten und/oder zweiten Wärmetauschers abgeführte, Wärme zuzuführen.

15. Vorrichtung nach einem der Ansprüche 11 bis 14, wobei im ersten Reaktionsgefäß (R1) mindestens ein erster Sensor, insbesondere ein erster pH-Sensor, ein erstes Refraktometer, ein erster Viskositätssensor, ein erster Dichtesensor, ein erster Inline-NMR-Sensor und/oder ein erstes Thermometer, zur Bestimmung einer Eigenschaft, insbesondere eines pH-Werts, eines Brechungsindex, einer Viskosität, einer Dichte, einer Zusammensetzung oder einer Temperatur, von Reaktionsflüssigkeit im ersten Reaktionsgefäß (R1) vorgesehen ist und/oder im zweiten Reaktionsgefäß (R2) mindestens ein zweiter Sensor, insbesondere ein zweiter pH-Sensor, ein zweites Refraktometer, ein zweiter Viskositätssensor, ein zweiter Dichtesensor, ein zweiter Inline-NMR-Sensor und/oder zweites Thermometer, zur Bestimmung einer Eigenschaft, insbesondere eines pH-Werts, eines Brechungsindex, einer Viskosität, einer Dichte, einer Zusammensetzung oder einer Temperatur, von Reaktionsflüssigkeit im zweiten Reaktionsgefäß (R2) vorgesehen ist.

## Claims

1. A process for the catalytic production of methanol from biomass by means of an electric current, wherein the biomass is a material comprising or consisting of at least one alpha-hydroxyaldehyde, at least one alpha-hydroxycarboxylic acid, at least one carbohydrate and/or at least one glycoside,
- wherein, in a first stage, O₂ and H₂ are produced from water by electrolysis using the current,
- wherein, in a second stage, the biomass is converted to formic acid in a first reaction vessel (R1) using an aqueous solution of a first catalyst; wherein the first catalyst, which is reduced during the catalytic reaction, is restored to its initial state by oxidation; wherein, for the oxidation of the reduced catalyst, the O₂ produced in the first stage is introduced into the solution in the first reaction vessel (R1), wherein the first catalyst is a poly-oxometalate ion of the general formula [PMoₓV_{y}O₄₀]ⁿ⁻, where 6 ≤ x ≤ 11, 1 ≤ y ≤ 6 and x + y = 12, is [WₓV_{y} O₁₉]ⁿ⁻, where x + y = 6, 3 ≤ x ≤ 5 and 1 ≤ y ≤ 3, or is [P₂WₓV_{y}O₆₂ ]ⁿ⁻, where x + y = 18, 12 ≤ x ≤ 17 and 1 ≤ y ≤ 6, or is a salt containing VO²⁺ or a salt containing [VO₃]⁻, where n, x and y are each an integer, the solution containing the formic acid formed therein being transferred to a second reaction vessel (R2), and methanol being added to the solution either during transfer to the second reaction vessel or within the second reaction vessel (R2), whereby the second reaction vessel (R2) is designed as a rectification column, optionally containing an acidic second catalyst which catalyses the esterification of the methanol with the formic acid, whereby the second catalyst is present in solid form as a bulk material or in liquid form as an acid, whereby in the second reaction vessel (R2), a reactive distillation is carried out and the methyl formate produced in the process is transferred to a tank (T),
- whereby, in a third stage, methyl formate is vaporised from the tank (T) and transferred to a third reaction vessel (R3), where it is hydrogenated with the H₂ from the first stage by means of a third catalyst which catalyses hydrogenation, whereby hydrogenolysis produces vapour-phase methanol, which is subsequently discharged from the third reaction vessel (R3) and cooled to such an extent that the methanol condenses,
- wherein, in a fourth stage, the residue of the reactive distillation remaining in the second reaction vessel (R2) from the second stage which contains the first catalyst is concentrated by water separation and fed to the first reaction vessel (R1),
wherein, in a first process mode, the current is used in the first stage to produce H₂, which is reacted with methyl formate in the third stage to form methanol; this methanol is transferred in its entirety to the second reaction vessel (R2) and reacted with formic acid in the second stage to form methyl formate; wherein, in a second process mode, the current is used in the first stage to produce H₂ in the first stage, which is reacted with methyl formate in the third stage to produce methanol, which is transferred to the second reaction vessel (R2) and reacted with formic acid in the second stage to produce methyl formate, and is also utilised in the first stage to produce additional H₂, whereby this additional H₂ is used in the third stage to produce additional methanol, which is discharged from the process, the process being operated alternately in the first and second process modes.

2. A process according to claim 1, wherein the methanol is added in the second stage in at least such a quantity that all the formic acid in the second reaction vessel is converted into methyl formate.

3. The process according to claim 2, wherein, at the start of the process, methanol is supplied from an external source in the second stage, and is supplied from an external source only for as long and in such a quantity as is necessary to ensure that, in the second stage as a whole, together with the methanol originating from the third stage and transferred into the second reaction vessel (R2) sufficient methanol is available to convert all the formic acid in the second reaction vessel (R2) to methyl formate.

4. A process according to any one of the preceding claims, wherein O₂ is separated from air or O₂-enriched air is produced by means of pressure-swing adsorption, and this O₂ or this O₂-enriched air is introduced in addition to the O₂ generated in the first stage into the solution in the first reaction vessel (R1) for the oxidation of the first catalyst reduced during the catalytic reaction and/or wherein, for the oxidation of the first catalyst reduced during the catalytic reaction, air is introduced in addition to the O₂ generated in the first stage into the solution in the first reaction vessel (R1).

5. A process according to any one of the preceding claims, wherein the second catalyst in liquid form is hydrochloric acid or sulphuric acid and the second catalyst in solid form is ZnO/ZrO₂ or a, in particular porous, supported, in particular silicon dioxide-supported, diarylammonium catalyst, an acidic cation exchanger, in particular an acidic zeolite or a polystyrene sulphonate, a supported, in particular silicon dioxide-, activated carbon- or porous glass-supported, heteropolyacid, in particular tungstophosphoric acid, 2-[1-[difluoro[(trifluoroethenyl)oxy]methyl]-1,2,2,2-tetrafluoroethoxy]-1,1,2,2-tetrafluoroethanesulfonic acid (Nafion^{®}) or mesoporous silica.

6. A process according to one of the preceding claims, wherein the third catalyst is a hydrogenation catalyst consisting of, or containing, at least one metal, in particular Pt, Pd, Rh, Ru, Ni, Co, Fe and Cu, in elemental or oxidised form, and present in solid form, in particular platinum black, platinum(IV) oxide, palladium black, colloidal palladium, palladium(II) oxide, palladium on calcium carbonate or barium sulphate, a Lindlar catalyst, a Nishimura catalyst, nickel on diatomaceous earth, a Raney nickel catalyst, copper chromite (CuCr₂O₄), an Adkins catalyst, a copper-zinc, copper-alumina or copper-bismuth catalyst, Raney cobalt, a Cu_{0.9}Al₂O₄ spinel-type catalyst, a CuO/Cr₂O₃ catalyst, a CuO/MgO/ZnO/Al₂O₃ catalyst, a Cu-ZnO₂ catalyst, a Cu-SiO₂ catalyst, a Raney copper catalyst or a copper-aluminium alloy catalyst.

7. A process according to any one of the preceding claims, wherein the separation of water in the fourth stage is carried out by evaporation, in particular flash evaporation, or by pervaporation, or wherein the separation of water is carried out by means of rectification or two-stage distillation, by means of which methanol still contained in the residue is also separated.

8. A process according to any of the preceding claims, wherein, in the first process mode, the H₂ produced in the first stage is partially supplemented or replaced by H₂ produced by the dehydrogenation of the methyl formate produced in the second stage using a dehydrogenation catalyst.

9. A process according to any one of the preceding claims, wherein water separated in the fourth stage is fed to the electrolysis in the first stage of and, optionally, is adjusted to the water in the first stage of the proceedings in terms of pH by adding a base or acid, and in terms of electrical resistance by separating the water through a semi-permeable membrane, in particular by reverse osmosis, and/or with regard to the ion concentration by passing it through one or more ion exchangers, before it is fed to the electrolysis in the first stage of the process.

10. A process according to any one of the preceding claims, wherein heat generated in the first reaction vessel (R1) in the second stage is dissipated by means of a first heat exchanger, and heat generated in the third reaction vessel (R3) in the third stage is dissipated by means of a second heat exchanger and supplied to the second reaction vessel (R2) by means of a third heat exchanger and/or, where necessary, to the water separation unit (D1) by means of a fourth heat exchanger.

11. Apparatus for carrying out the process according to one of the preceding claims, wherein the apparatus comprises
- an electrolysis unit (E1),
- an oxidation unit comprising a first reaction vessel (R1) and an aqueous solution of the first catalyst, as specified in claim 1, contained therein,
- a reactive distillation unit comprising a second reaction vessel (R2) designed as a rectification column, which optionally contains an acidic second catalyst present in liquid form as an acid or in solid form as a bulk material,
- a tank (T) for holding methyl formate,
- an evaporator (V),
- a hydrogenation unit comprising a third reaction vessel (R3), in which a third catalyst catalysing a hydrogenation is contained,
- a cooling and condensation unit (K),
- a further tank (Tw) and
- a water separation unit (D1),
whereby a first gas line (7) for transferring O₂ from the electrolysis unit to the first reaction vessel (R1), a second gas line (21) for transferring H₂ from the electrolysis unit to the third reaction vessel (R3), a first liquid line (13) for transferring reaction liquid from the first reaction vessel (R1) to the second reaction vessel (R2), a second liquid line (27) for transferring reaction liquid from the second reaction vessel (R2) to the water separation unit (D1), a third liquid line (29) for transferring reaction liquid from the water separation unit (D1) to the first reaction vessel (R1), a fourth liquid line (5) for transferring water from the water separation unit (D1) to the electrolysis unit (E1), a fifth liquid line (15) for transferring methyl formate from the second reaction vessel (R2) to the tank (T), an eighth liquid line (17) for transferring methyl formate from the tank (T) to the evaporator (V), a fourth gas line (19) for transferring vaporised methyl formate into the third reaction vessel (R3), a fifth gas line (22) for transferring vapour-phase methanol from the third reaction vessel (R3) into the cooling and condensation unit (K), a sixth liquid line (23) for transferring methanol liquefied in the cooling and condensation unit (K) from the cooling and condensation unit (K) to the additional tank (Tw), a ninth liquid line (24) for discharging the methanol from the additional tank (Tw), a seventh liquid line (25) for transferring methanol from the cooling and condensation unit (K), the sixth liquid line (23), the additional tank (Tw) or the ninth liquid line (24) into the second reaction vessel (R2) or into the first liquid line (13), and a control unit are provided, wherein the control unit controls,
- that a constant volume flow of methanol, required for the conversion of all the formic acid produced and transferred to the second reaction vessel (R2) into methyl formate, is fed into the second reaction vessel (R2) via the seventh liquid line (25), and that at least a first quantity of H₂ is always generated per minute in the electrolysis unit (E1) and fed to the third reaction vessel (R3) via the second gas line (21), such that sufficient methanol is produced to maintain the constant volume flow of methanol, whereby a quantity of methyl formate is always fed from the tank (T) into the evaporator (V) and from there into the third reaction vessel (R3) such that all the H₂ transferred to the third reaction vessel (R3) is consumed in the hydrogenation of the methyl formate,
- that the methanol produced in the third reaction vessel (R3) is fed in its entirety at a constant flow rate via the seventh liquid line (25) into the second reaction vessel (R2) when the process is carried out in a first operating mode, and
- that, in the electrolysis unit (E1), a second quantity of H₂ is additionally produced per minute and fed to the third reaction vessel (R3) via the second gas line (21) in addition to the first quantity of H₂ per minute, and a resulting proportion of the methanol produced which is not required to maintain the constant volume flow is discharged from the process via the ninth liquid line (24) when the process is carried out in a second operating mode.

12. An apparatus according to claim 11, wherein a compressor is provided in each of the first gas line (7) and/or the second gas line (21) and/or the fourth gas line (19) and/or the fifth gas line (22) for conveying or increasing the pressure of the gas/vapour contained therein, and/or wherein at least in the first (13), second (27), third (29), fourth (5), fifth (15), sixth (23), seventh (25), eighth (17) and/or ninth liquid line (24) is in each case provided with a pump for conveying the medium contained therein.

13. An apparatus according to claim 11 or 12, wherein a gassing unit, in particular a static mixer or an agitator comprising a gas injection device or an agitator in combination with a gas injection device, is provided in the first reaction vessel (R1) for the gassing the first catalyst reduced in a catalytic reaction with O₂ and/or wherein a pressure-swing adsorption unit is provided for the enrichment of O₂ in, or the separation of O₂ from, air, wherein a third gas line is provided which connects the pressure-swing adsorption unit to the first gas line (7) or the first reaction vessel, in order to feed the O₂ enriched air or the O₂ into the first gas line (7) or the first reaction vessel (R1).

14. Apparatus according to any one of claims 11 to 13, wherein a first heat exchanger is provided in the first reaction vessel (R1) to extract and dissipate heat generated in the second stage of the process according to any one of claims 1 to 10 from the solution contained therein, and/or a second heat exchanger is provided in the third reaction vessel (R3) to extract and dissipate heat generated in the third stage of the process according to any one of claims 1 to 10, and/or a third heat exchanger or a first heater is provided in the second reaction vessel (R2) to supply heat, in particular heat dissipated by means of the first and/or second heat exchanger, to the reactive distillation in the second stage of the process according to any one of claims 1 to 10 and/or a fourth heat exchanger or a second heater is provided in the water separation unit (D1) to supply heat, in particular heat dissipated by means of the first and/or second heat exchanger, to the water separation unit (D1), where necessary,.

15. Apparatus according to any one of claims 11 to 14, wherein at least one first sensor, in particular a first pH sensor, a first refractometer, a first viscosity sensor, a first density sensor, a first in-line NMR sensor and/or a first thermometer, is provided in the first reaction vessel (R1) for determining a property, in particular a pH value, a refractive index, a viscosity, a density, a composition or a temperature, of the reaction liquid in the first reaction vessel (R1), and/or at least a second sensor, in particular a second pH sensor, a second refractometer, a second viscosity sensor, a second density sensor, a second in-line NMR sensor and/or a second thermometer, is provided in the second reaction vessel (R2) for determining a property, in particular a pH value, a refractive index, a viscosity, a density, a composition or a temperature, of the reaction liquid in the second reaction vessel (R2).

## Revendications

1. Procédé de génération catalytique de méthanol à partir de biomasse au moyen de courant électrique, dans lequel la biomasse est un matériau qui comprend au moins un alpha-hydroxyaldéhyde, au moins un acide alpha-hydroxycarboxylique, au moins un hydrate de carbone et/ou au moins un glycoside ou se compose de ceux-ci,
- dans lequel de l'O₂ et du H₂ sont générés dans une première étape à partir d'eau par électrolyse au moyen du courant,
- dans lequel la biomasse est convertie en acide formique dans une deuxième étape au moyen d'une solution aqueuse d'un premier catalyseur dans un premier récipient réactionnel (R1), dans lequel le premier catalyseur réduit lors de la réaction catalytique est remis dans son état de départ par oxydation, dans lequel l'O₂ généré dans la première étape est introduit dans la solution dans le premier récipient réactionnel (R1) pour l'oxydation du catalyseur réduit, dans lequel le premier catalyseur est un ion polyoxométallate de la formule générale [PMoₓV_{y}O₄₀]ⁿ⁻¹, dans laquelle 6 ≤ x ≤ 11, 1 ≤ y ≤ 6 et x + y = 12, est [WₓV_{y}O₁₉]ⁿ⁻, dans laquelle x + y = 6, 3 ≤ x ≤ 5 et 1 ≤ y ≤ 3, ou est [P₂WₓV_{y}O₆₂]ⁿ⁻, dans laquelle x + y = 18, 12 ≤ x ≤ 17 et 1 ≤ y ≤ 6, ou est un sel contenant VO²⁺ ou un sel contenant [VO₃]⁻, dans lesquelles n, x et y sont chacun un nombre entier, dans lequel la solution avec l'acide formique apparu en son sein est transférée dans un deuxième récipient réactionnel (R2), dans lequel du méthanol est ajouté à la solution lors du transfert dans le deuxième récipient réactionnel ou dans le deuxième récipient réactionnel (R2), dans lequel le deuxième récipient réactionnel (R2) est réalisé en tant que colonne de rectification dans laquelle est contenu en option un deuxième catalyseur acide catalysant une estérification du méthanol avec l'acide formique, dans lequel le deuxième catalyseur est présent sous forme solide en vrac ou sous forme liquide en tant qu'acide, dans lequel une distillation réactive est réalisée dans le deuxième récipient réactionnel (R2) et le formiate de méthyle apparaissant ce faisant est transféré dans un réservoir (T),
- dans lequel du formiate de méthyle provenant du réservoir (T) est évaporé dans une troisième étape et transféré dans un troisième récipient réactionnel (R3), et y est hydrogéné avec le H₂ provenant de la première étape au moyen d'un troisième catalyseur catalysant une hydrogénation, dans lequel du méthanol sous forme de vapeur apparaît par hydrogénolyse, lequel est ensuite dévié du troisième récipient réactionnel (R3) et refroidi jusqu'à ce que le méthanol se condense,
- dans lequel le résidu de la distillation réactive restant dans la deuxième étape dans le deuxième récipient réactionnel (R2), contenant le premier catalyseur, est concentré dans une quatrième étape par séparation d'eau et acheminé au premier récipient réactionnel (R1),
dans lequel le courant est utilisé dans un premier mode de procédé pour la génération de H₂ dans la première étape, lequel est converti dans la troisième étape avec le formiate de méthyle en méthanol qui est transféré entièrement dans le deuxième récipient réactionnel (R2) et converti dans la deuxième étape avec l'acide formique en formiate de méthyle, dans lequel le courant est utilisé dans un second mode de procédé pour la génération de H₂ dans la première étape, lequel est converti dans la troisième étape avec le formiate de méthyle en méthanol qui est transféré dans le deuxième récipient réactionnel (R2) et converti dans la deuxième étape avec l'acide formique en formiate de méthyle, et est en outre utilisé dans la première étape pour la génération de H₂ supplémentaire, dans lequel ce H₂ supplémentaire est utilisé dans la troisième étape pour la génération de méthanol supplémentaire qui est évacué du procédé, dans lequel le procédé est exploité en alternance dans le premier et dans le second mode de procédé.

2. Procédé selon la revendication 1, dans lequel le méthanol dans la deuxième étape est ajouté au moins en une quantité telle que l'acide formique complet est converti dans le deuxième récipient réactionnel avec lui en formiate de méthyle.

3. Procédé selon la revendication 2, dans lequel du méthanol est acheminé de l'extérieur dans la deuxième étape au début d'une réalisation du procédé, dans lequel il n'est acheminé de l'extérieur qu'aussi longtemps et uniquement dans une telle quantité que nécessaire afin que suffisamment de méthanol soit à disposition au total dans la deuxième étape conjointement au méthanol provenant de la troisième étape et transféré dans le deuxième récipient réactionnel (R2) pour convertir avec lui l'acide formique complet dans le deuxième récipient réactionnel (R2) en formiate de méthyle.

4. Procédé selon une des revendications précédentes, dans lequel de l'O₂ est séparé de l'air au moyen d'adsorption par variation de pression ou un air enrichi en O₂ est généré et cet O₂ ou cet air enrichi en O₂ est introduit dans la solution dans le premier récipient réactionnel (R1) pour l'oxydation du premier catalyseur réduit lors de la réaction catalytique outre l'O₂ généré dans la première étape et/ou dans lequel de l'air est introduit dans la solution dans le premier récipient réactionnel (R1) pour l'oxydation du premier catalyseur réduit lors de la réaction catalytique outre l'O₂ généré dans la première étape.

5. Procédé selon une des revendications précédentes, dans lequel le deuxième catalyseur sous forme liquide est de l'acide chlorhydrique ou de l'acide sulfurique et le deuxième catalyseur sous forme solide est ZnO/ZrO₂ ou un catalyseur de diarylammonium notamment poreux, supporté, notamment supporté par du dioxyde de silicium, un échangeur de cations acide, notamment une zéolite acide ou un sulfonate de polystyrène, un hétéropolyacide supporté, notamment supporté par du dioxyde de silicium, du charbon actif ou du verre poreux, notamment de l'acide tungstatophosphorique, de l'acide 2-[1-[difluoro[(trifluoroéthényl)oxy]méthyl]-1,2,2,2-tétrafluoroéthoxy]-1,1,2,2-tétrafluoroéthanesulfonqiue (Nafion^{®}) ou de la silice mésoporeuse.

6. Procédé selon une des revendications précédentes, dans lequel le troisième catalyseur est un catalyseur d'hydrogénation se composant d'au moins un métal, notamment Pt, Pd, Rh, Ru, Ni, Co, Fe et Cu, sous forme élémentaire ou oxydée ou contenant un tel métal et présent sous forme solide, notamment du noir de platine, de l'oxyde de platine(IV), du noir de palladium, du palladium colloïdal, de l'oxyde de palladium(II), du palladium sur du carbonate de calcium ou du sulfate de baryum, un catalyseur de Lindlar, un catalyseur de Nishimura, du nickel sur du kieselguhr, un catalyseur de nickel de Raney, du chromite de cuivre (CuCr₂O₄), un catalyseur d'Adkins, un catalyseur de cuivre-zinc, de cuivre-alumine ou de cuivre-bismuth, du cobalt de Raney, un catalyseur de type spinelle de Cu_{0,9}Al₂O₄, un catalyseur de CuO/Cr₂O₃, un catalyseur de CuO/MgO/ZnO/Al₂O₃, un catalyseur de Cu-ZnO₂, un catalyseur de Cu-SiO₂, un catalyseur de cuivre de Raney ou un catalyseur en alliage de cuivre-aluminium.

7. Procédé selon une des revendications précédentes, dans lequel la séparation d'eau dans la quatrième étape s'effectue par évaporation, notamment une évaporation de détente, ou par pervaporation ou dans lequel la séparation d'eau s'effectue au moyen d'une rectification ou distillation en deux étapes, au moyen de laquelle une séparation de méthanol encore contenu dans le résidu s'effectue également.

8. Procédé selon une des revendications précédentes, dans lequel le H₂ généré dans la première étape est partiellement complété ou remplacé par du H₂ dans le premier mode de procédé, lequel est généré par déshydrogénation du formiate de méthyle généré dans la deuxième étape au moyen d'un catalyseur de déshydrogénation.

9. Procédé selon une des revendications précédentes, dans lequel de l'eau séparée dans la quatrième étape est acheminée à l'électrolyse dans la première étape et est adaptée en option à l'eau dans la première étape du procédé en ce qui concerne la valeur de pH par addition d'une base ou d'un acide, en ce qui concerne la résistance électrique au moyen de séparation de l'eau à travers une membrane semi-perméable, notamment par une osmose inverse, et/ou en ce qui concerne la concentration ionique par passage à travers un ou plusieurs échangeurs d'ions avant qu'elle ne soit acheminée à l'électrolyse dans la première étape.

10. Procédé selon une des revendications précédentes, dans lequel de la chaleur apparaissant dans le premier récipient réactionnel (R1) dans la deuxième étape est évacuée au moyen d'un premier échangeur de chaleur et de la chaleur apparaissant dans le troisième récipient réactionnel (R3) dans la troisième étape est évacuée au moyen d'un deuxième échangeur de chaleur et acheminée au deuxième récipient réactionnel (R2) au moyen d'un troisième échangeur de chaleur et/ou de l'unité de séparation d'eau (D1), le cas échéant, au moyen d'un quatrième échangeur de chaleur.

11. Dispositif pour la réalisation du procédé selon une des revendications précédentes, dans lequel le dispositif comprend
- une unité d'électrolyse (E1),
- une unité d'oxydation avec un premier récipient réactionnel (R1) et une solution aqueuse du premier catalyseur contenue en son sein, tel qu'il est spécifié dans la revendication 1,
- une unité de distillation réactive avec un deuxième récipient réactionnel (R2) réalisé en tant que colonne de rectification dans lequel un deuxième catalyseur acide présent sous forme liquide en tant qu'acide ou sous forme solide en vrac est contenu en option,
- un réservoir (T) pour la réception de formiate de méthyle,
- un évaporateur (V),
- une unité d'hydrogénation avec un troisième récipient réactionnel (R3) dans lequel un troisième catalyseur catalysant une hydrogénation est contenu,
- une unité de refroidissement et de condensation (K),
- un autre réservoir (Tw), et
- une unité de séparation d'eau (D1),
dans lequel sont prévues une première conduite gazeuse (7) pour le transfert d'O₂ de l'unité d'électrolyse au premier récipient réactionnel (R1), une deuxième conduite gazeuse (21) pour le transfert de H₂ de l'unité d'électrolyse au troisième récipient réactionnel (R3), une première conduite fluidique (13) pour le transfert de fluide réactionnel du premier récipient réactionnel (R1) dans le deuxième récipient réactionnel (R2), une deuxième conduite fluidique (27) pour le transfert de fluide réactionnel du deuxième récipient réactionnel (R2) dans l'unité de séparation d'eau (D1), une troisième conduite fluidique (29) pour le transfert de fluide réactionnel de l'unité de séparation d'eau (D1) dans le premier récipient réactionnel (R1), une quatrième conduite fluidique (5) pour le transfert d'eau de l'unité de séparation d'eau (D1) dans l'unité d'électrolyse (E1), une cinquième conduite fluidique (15) pour le transfert de formiate de méthyle du deuxième récipient réactionnel (R2) dans le réservoir (T), une huitième conduite fluidique (17) pour le transfert de formiate de méthyle du réservoir T dans l'évaporateur (V), une quatrième conduite gazeuse (19) pour le transfert de formiate de méthyle évaporé dans le troisième récipient réactionnel (R3), une cinquième conduite gazeuse (22) pour le transfert de méthanol sous forme de vapeur du troisième récipient réactionnel (R3) dans l'unité de refroidissement et de condensation (K), une sixième conduite fluidique (23) pour le transfert de méthanol fluidifié dans l'unité de refroidissement et de condensation K de l'unité de refroidissement et de condensation (K) dans l'autre réservoir (Tw), une neuvième conduite fluidique (24) pour la déviation du méthanol de l'autre réservoir (Tw), une septième conduite fluidique (25) pour le transfert de méthanol de l'unité de refroidissement et de condensation (K), de la sixième conduite fluidique (23), de l'autre réservoir (Tw) ou de la neuvième conduite fluidique (24) dans le deuxième récipient réactionnel (R2) ou dans la première conduite fluidique (13) et une unité de régulation, dans lequel l'unité de régulation commande
- qu'un courant volumique constant de méthanol nécessaire pour la conversion de l'acide formique complet généré et transféré dans le deuxième récipient réactionnel (R2) en formiate de méthyle est dirigé par le biais de la septième conduite fluidique (25) dans le deuxième récipient réactionnel (R2) et au moins une telle première quantité de H₂ par minute est toujours générée dans l'unité d'électrolyse (E1) et acheminée au troisième récipient réactionnel (R3) par le biais de la deuxième conduite gazeuse (21) que suffisamment de méthanol est généré pour pouvoir maintenir le courant volumique constant de méthanol, dans lequel une telle quantité de formiate de méthyle est toujours dirigée du réservoir (T) dans l'évaporateur (V) et de là dans le troisième récipient réactionnel (R3), que le H₂ complet transféré dans le troisième récipient réactionnel (R3) est consommé pour une hydrogénation du formiate de méthyle,
- que le méthanol généré dans le troisième récipient réactionnel (R3) est dirigé avec le courant volumique constant entièrement par le biais de la septième conduite fluidique (25) dans le deuxième récipient réactionnel (R2) lorsque le procédé est réalisé dans un premier mode de procédé et
- qu'en outre, une seconde quantité de H₂ par minute est générée dans l'unité d'électrolyse (E1) et acheminée au troisième récipient réactionnel (R3) par le biais de la deuxième conduite gazeuse (21) outre la première quantité de H₂ par minute et une proportion en résultant, non nécessaire pour le maintien du courant volumique constant du méthanol généré est évacuée du procédé par le biais de la neuvième conduite fluidique (24) lorsque le procédé est réalisé dans un second mode de procédé.

12. Dispositif selon la revendication 11, dans lequel un compresseur pour le transport ou l'augmentation d'une pression de gaz/vapeur contenu(e) respectivement en son sein est prévu dans la première conduite gazeuse (7) et/ou la deuxième conduite gazeuse (21) et/ou la quatrième conduite gazeuse (19) et/ou la cinquième conduite gazeuse (22) et/ou dans lequel une pompe pour le transport du milieu contenu respectivement en son sein est à chaque fois prévue au moins dans la première (13), deuxième (27), troisième (29), quatrième (5), cinquième (15), sixième (23), septième (25), huitième (17) et/ou neuvième conduite fluidique (24).

13. Dispositif selon la revendication 11 ou 12, dans lequel une unité de gazage, notamment un mélangeur statique ou un agitateur comprenant un dispositif d'alimentation en gaz ou un agitateur en combinaison avec un dispositif d'alimentation en gaz, est prévue dans le premier récipient réactionnel (R1) pour le gazage du premier catalyseur réduit lors d'une réaction catalytique avec de l'O₂ et/ou dans lequel un appareil d'adsorption par variation de pression est prévu pour l'enrichissement en O₂ dans ou l'isolation d'O₂ de l'air, dans lequel une troisième conduite gazeuse est prévue, laquelle connecte l'appareil d'adsorption par variation de pression à la première conduite gazeuse (7) ou au premier récipient réactionnel pour injecter l'air enrichi en O₂ ou l'O₂ dans la première conduite gazeuse (7) ou le premier récipient réactionnel (R1).

14. Dispositif selon une des revendications 11 à 13, dans lequel un premier échangeur de chaleur est prévu dans le premier récipient réactionnel (R1) pour extraire et évacuer de la chaleur apparaissant de la solution contenue en son sein dans la deuxième étape du procédé selon une des revendications 1 à 10 et/ou un deuxième échangeur de chaleur est prévu dans le troisième récipient réactionnel (R3) pour extraire et évacuer de la chaleur apparaissant dans la troisième étape du procédé selon une des revendications 1 à 10 et/ou un troisième échangeur de chaleur ou un premier chauffage est prévu dans le deuxième récipient réactionnel (R2) pour acheminer de la chaleur, notamment évacuée au moyen du premier et/ou deuxième échangeur de chaleur, à la distillation réactive dans la deuxième étape du procédé selon une des revendications 1 à 10 et/ou un quatrième échangeur de chaleur ou un second chauffage est prévu dans l'unité de séparation d'eau (D1) pour acheminer, le cas échéant, de la chaleur, évacuée notamment au moyen du premier et/ou deuxième échangeur de chaleur à l'unité de séparation d'eau (D1).

15. Dispositif selon une des revendications 11 à 14, dans lequel au moins un premier capteur, notamment un premier pH-mètre, un premier réfractomètre, un premier capteur de viscosité, un premier capteur de densité, un premier capteur RMN en ligne et/ou un premier thermomètre, est prévu dans le premier récipient réactionnel (R1) pour la détermination d'une propriété, notamment d'une valeur de pH, d'un indice de réfraction, d'une viscosité, d'une densité, d'une composition ou d'une température, de fluide réactionnel dans le premier récipient réactionnel (R1) et/ou au moins un second capteur, notamment un second pH-mètre, un second réfractomètre, un second capteur de viscosité, un second capteur de densité, un second capteur RMN en ligne et/ou second thermomètre, est prévu dans le deuxième récipient réactionnel (R2) pour la détermination d'une propriété, notamment d'une valeur de pH, d'un indice de réfraction, d'une viscosité, d'une densité, d'une composition ou d'une température, de fluide réactionnel dans le deuxième récipient réactionnel (R2).
